# EUROPEAN PATENT SPECIFICATION

(11) **EP 2 399 598 B1**
(45) Date of publication and mention of the grant of the patent: **06.08.2014**
(21) Application number: 10167496.8
(22) Date of filing: 28.06.2010
(51) Int. Cl.: A61K 38/00, A61K 48/00, C07K 14/47, G01N 33/574, A61P 11/00, A61P 35/00, C12N 15/00

(54) **Blockade of CCL18 signaling via CCR6 as a therapeutic option in fibrotic diseases and cancer**
Blockade der CCL18-Signalisierung über CCR6 als therapeutische Option bei fibrotischen Erkrankungen und Krebs
Blocage de la signalisation CCL18 au moyen de CCR6 en tant qu'option thérapeutique dans des maladies fibrogènes et le cancer

(43) Date of publication of application: 28.12.2011
(73) Proprietor: Universitätsklinikum Freiburg, 79106 Freiburg (DE)
(72) Inventor: Zissel, Gernot, 79114 Freiburg (DE); Müller-Quernheim, Joachim, 79199 Kirchzarten (DE); Prasse, Antje, 79104 Freiburg (DE)
(74) Representative: Bühler, Dirk

(56) References cited:
- EP-A2- 1 422 239
- WO-A2-00/55180
- WO-A2-01/32874
- WO-A2-2005/015206
- WO-A2-2008/054764
- US-A1- 2004 161 425
- Corinna Maier: "Expressionsanalyse des Chemokinrezeptors CCR6 auf Fibroblasten"[Online] 12 November 2008 (2008-11-12), XP002603043 Retrieved from the Internet: URL:http://www.freidok.uni-freiburg.de/vol ltexte/6017/pdf/DrFertigSW2008.pdf> [retrieved on 2010-09-23]
- Zissel: "Inhibition der CCL18-induzierten Zellaktivierung durch ein CCL18-Inhibitor Peptid"[Online] 11 February 2010 (2010-02-11), XP002603044 Retrieved from the Internet: URL:http://www.uniklinik-freiburg.de/pneum ologie/live/forschung/Doktorarbeiten/CCL18 -Inhibitor.pdf> [retrieved on 2010-09-23]
- PARDO A ET AL: "CCL18/DC-C5-1/PARC UP-REGULATION IN HYPERSENSITIVITY PNEUMONITIS" JOURNAL OF LEUKOCYTE BIOLOGY, FEDERATION OF AMERICAN SOCIETIES FOR EXPERIMENTAL BIOLOGY, US, vol. 70, no. 4, 1 October 2001 (2001-10-01), pages 610-616, XP001057229 ISSN: 0741-5400

### SEQUENCE LISTING

<110> UNIVERSITÄTSKLINIKUM FREIBURG
<120> Blockade of CCL18 signaling via CCR6 as a Therapeutic option in Fibrotic Diseases and Cancer
<130> U7130/DB
<160> 19
<170> PatentIn version 3.3
<210> 1
   <211> 29
   <212> PRT
   <213> Artificial
<220>
   <223> peptide inhibitor of CCR6
<400> 1
   Glu Asp Cys Cys Leu Val Tyr Thr Ser Trp Gln Ile His Pro Lys Phe
   1               5                   10                  15
   Ile Val Asp Tyr Ser Glu Thr Ser Pro Gln Cys Pro Lys
               20                  25
<210> 2
   <211> 29
   <212> PRT
   <213> Artificial
<220>
   <223> peptide inhibitor of CCR6
<400> 2
   Glu Leu Cys Cys Leu Val Tyr Thr Ser Trp Gln Ile Pro Gln Lys Phe
   1               5                   10                  15
   Ile Val Asp Tyr Ser Glu Thr Ser Pro Gln Cys Pro Lys
               20                  25
<210> 3
   <211> 31
   <212> PRT
   <213> Artificial
<220>
   <223> peptide inhibitor of CCR6
<400> 3
   Phe Asp Cys Cys Leu Gly Tyr Thr Asp Arg Ile Leu His Pro Lys Phe
   1               5                   10                  15
   Ile Val Gly Phe Thr Arg Gln Leu Ala Asn Glu Gly Cys Asp Ile
               20                  25                  30
<210> 4
   <211> 87
   <212> DNA
   <213> Artificial
<220>
   <223> polynucleotide sequence encoding peptide according to SEQ ID NO.: 1
<400> 4
   gaggactgct gcctcgtcta tacctcctgg cagattcacc caaagttcat agttgactat     60
   tctgaaacca gcccccagtg ccccaag                                         87
<210> 5
   <211> 87
   <212> DNA
   <213> Artificial
<220>
   <223> polynucleotide sequence encoding polypeptide according to SEQ ID NO.: 2
<400> 5
   gagctctgct gcctcgtcta tacctcctgg cagattccac aaaagttcat agttgactat     60
   tctgaaacca gcccccagtg ccccaag                                         87
<210> 6
   <211> 93
   <212> DNA
   <213> Artificial
<220>
   <223> polynucleotide sequence encoding polypeptide according to SEQ ID NO.: 3
<400> 6
   tttgactgct gtcttggata cacagaccgt attcttcatc ctaaatttat tgtgggcttc     60
   acacggcagc tggccaatga aggctgtgac atc                                  93
<210> 7
   <211> 3216
   <212> DNA
   <213> Homo sapiens
<400> 7
   agtgtatggg tgaaggaggc agcagtgtgg ccggagagga gagctgggct gggagcacag     60
   gaaggtcccc aggactctgt ggtcatcagt aagagagggc ccacgtgtat atgctggtga    120
   acagaaatgt caaccttttc aaagtctgac atttaagaga aaaaactgtg gctgttggtt    180
   tgtggaacag acagctcctt ctttattgag tcacctctac tttcctgcta ccgctgcctg    240
   tgagctgaag gggctgaacc atacactcct ttttctacaa ccagcttgca ttttttctgc    300
   ccacaatgag cggggaatca atgaatttca gcgatgtttt cgactccagt gaagattatt    360
   ttgtgtcagt caatacttca tattactcag ttgattctga gatgttactg tgctccttgc    420
   aggaggtcag gcagttctcc aggctatttg taccgattgc ctactccttg atctgtgtct    480
   ttggcctcct ggggaatatt ctggtggtga tcacctttgc tttttataag aaggccaggt    540
   ctatgacaga cgtctatctc ttgaacatgg ccattgcaga catcctcttt gttcttactc    600
   tcccattctg ggcagtgagt catgccaccg gtgcgtgggt tttcagcaat gccacgtgca    660
   agttgctaaa aggcatctat gccatcaact ttaactgcgg gatgctgctc ctgacttgca    720
   ttagcatgga ccggtacatc gccattgtac aggcgactaa gtcattccgg ctccgatcca    780
   gaacactacc gcgcagcaaa atcatctgcc ttgttgtgtg ggggctgtca gtcatcatct    840
   ccagctcaac ttttgtcttc aaccaaaaat acaacaccca aggcagcgat gtctgtgaac    900
   ccaagtacca gactgtctcg gagcccatca ggtggaagct gctgatgttg gggcttgagc     960
   tactctttgg tttctttatc cctttgatgt tcatgatatt ttgttacacg ttcattgtca   1020
   aaaccttggt gcaagctcag aattctaaaa ggcacaaagc catccgtgta atcatagctg   1080
   tggtgcttgt gtttctggct tgtcagattc ctcataacat ggtcctgctt gtgacggctg   1140
   caaatttggg taaaatgaac cgatcctgcc agagcgaaaa gctaattggc tatacgaaaa   1200
   ctgtcacaga agtcctggct ttcctgcact gctgcctgaa ccctgtgctc tacgctttta   1260
   ttgggcagaa gttcagaaac tactttctga agatcttgaa ggacctgtgg tgtgtgagaa   1320
   ggaagtacaa gtcctcaggc ttctcctgtg ccgggaggta ctcagaaaac atttctcggc   1380
   agaccagtga gaccgcagat aacgacaatg cgtcgtcctt cactatgtga tagaaagctg   1440
   agtctcccta aggcatgtgt gaaacatact catagatgtt atgcaaaaaa aagtctatgg   1500
   ccaggtatgc atggaaaatg tgggaattaa gcaaaatcaa gcaagcctct ctcctgcggg   1560
   acttaacgtg ctcatgggct gtgtgatctc ttcagggtgg ggtggtctct gataggtagc   1620
   attttccagc actttgcaag gaatgttttg tagctctagg gtatatatcc gcctggcatt   1680
   tcacaaaaca gcctttggga aatgctgaat taaagtgaat tgttgacaaa tgtaaacatt   1740
   ttcagaaata ttcatgaagc ggtcacagat cacagtgtct tttggttaca gcacaaaatg   1800
   atggcagtgg tttgaaaaac taaaacagaa aaaaaaatgg aagccaacac atcactcatt   1860
   ttaggcaaat gtttaaacat ttttatctat cagaatgttt attgttgctg gttataagca   1920
   gcaggattgg ccggctagtg tttcctctca tttccctttg atacagtcaa caagcctgac   1980
   cctgtaaaat ggaggtggaa agacaagctc aagtgttcac aacctggaag tgcttcggga   2040
   agaaggggac aatggcagaa caggtgttgg tgacaattgt caccaattgg ataaagcagc   2100
   tcaggttgta gtgggccatt aggaaactgt cggtttgctt tgatttccct gggagctgtt   2160
   ctctgtcgtg agtgtctctt gtctaaacgt ccattaagct gagagtgcta tgaagacagg   2220
   atctagaata atcttgctca cagctgtgct ctgagtgcct agcggagttc cagcaaacaa   2280
   aatggactca agagagattt gattaatgaa tcgtaatgaa gttggggttt attgtacagt   2340
   ttaaaatgtt agatgttttt aattttttaa ataaatggaa tacttttttt ttttttttaa   2400
   agaaagcaac tttactgaga caatgtagaa agaagttttg ttccgtttct ttaatgtggt   2460
   tgaagagcaa tgtgtggctg aagacttttg ttatgaggag ctgcagatta gctaggggac   2520
   agctggaatt atgctggctt ctgataatta ttttaaaggg gtctgaaatt tgtgatggaa   2580
   tcagatttta acagctctct tcaatgacat agaaagttca tggaactcat gtttttaaag   2640
   ggctatgtaa atatatgaac attagaaaaa tagcaacttg tgttacaaaa atacaaacac   2700
   atgttaggaa ggtactgtca tgggctaggc atggtggctc acacctgtaa tcccagcatt   2760
   ttgggaagct aagatgggtg gatcacttga ggtcaggagt ttgagaccag cctggccaac   2820
   atggcgaaac ccctctctac taaaaataca aaaatttgcc aggcgtggtg gcgggtgcct   2880
   gtaatcccag ctacttggga ggctgaggca agagaatcgc ttgaacccag gaggcagagg   2940
   ttgcagtgag ccgagatcgt gccattgcac tccagcctgg gtgacaaagc gagactccat   3000
   ctcaaaaaaa aaaaaaaaaa aaaaggaaag aactgtcatg taaacatacc aacatgttta   3060
   aacctgacaa tggtgttatt tgaaacttta tattgttctt gtaagcttta actatatctc   3120
   tctttaaaat gcaaaataat gtcttaagat tcaaagtctg tatttttaaa gcatggcttt   3180
   ggctttgcaa aataaaaaat gtgttttgta catgaa                             3216
<210> 8
   <211> 3479
   <212> DNA
   <213> Homo sapiens
<400> 8
   aactcacacg gcctcttgca aacgttccca aatcttccca gtcggcttgc agagactcct      60
   tgctcccagg agataaccag gtaaaggagt atgaaagttt gggtacaaac tcattgctgc     120
   aaattgaaaa ccatgcaaag gctgtcttcc tctggggagt tcaatgcctc tctttttctt     180
   atcactttac cattggttgg actttgattc cagggatcct acgattactc aataccctac     240
   aggatataca tggttaacca tttgcatttg ggcaaatagg cgttactttt caataggaag     300
   tggcaatcca gaacttgctt ttgggcaatt ctagtagctc accgcttttt tcttaatgac     360
   tgctagaagc tgcatcttat tgacagatgg tcatcacatt ggtgagctgg agtcatcaga     420
   ttgtggggcc cggagtgagg ctgaagggag tggatcagag cactgcctga gagtcacctc     480
   tactttcctg ctaccgctgc ctgtgagctg aaggggctga accatacact cctttttcta     540
   caaccagctt gcattttttc tgcccacaat gagcggggaa tcaatgaatt tcagcgatgt      600
   tttcgactcc agtgaagatt attttgtgtc agtcaatact tcatattact cagttgattc     660
   tgagatgtta ctgtgctcct tgcaggaggt caggcagttc tccaggctat ttgtaccgat     720
   tgcctactcc ttgatctgtg tctttggcct cctggggaat attctggtgg tgatcacctt     780
   tgctttttat aagaaggcca ggtctatgac agacgtctat ctcttgaaca tggccattgc     840
   agacatcctc tttgttctta ctctcccatt ctgggcagtg agtcatgcca ccggtgcgtg     900
   ggttttcagc aatgccacgt gcaagttgct aaaaggcatc tatgccatca actttaactg     960
   cgggatgctg ctcctgactt gcattagcat ggaccggtac atcgccattg tacaggcgac    1020
   taagtcattc cggctccgat ccagaacact accgcgcagc aaaatcatct gccttgttgt    1080
   gtgggggctg tcagtcatca tctccagctc aacttttgtc ttcaaccaaa aatacaacac    1140
   ccaaggcagc gatgtctgtg aacccaagta ccagactgtc tcggagccca tcaggtggaa    1200
   gctgctgatg ttggggcttg agctactctt tggtttcttt atccctttga tgttcatgat    1260
   attttgttac acgttcattg tcaaaacctt ggtgcaagct cagaattcta aaaggcacaa    1320
   agccatccgt gtaatcatag ctgtggtgct tgtgtttctg gcttgtcaga ttcctcataa   1380
   catggtcctg cttgtgacgg ctgcaaattt gggtaaaatg aaccgatcct gccagagcga   1440
   aaagctaatt ggctatacga aaactgtcac agaagtcctg gctttcctgc actgctgcct   1500
   gaaccctgtg ctctacgctt ttattgggca gaagttcaga aactactttc tgaagatctt   1560
   gaaggacctg tggtgtgtga gaaggaagta caagtcctca ggcttctcct gtgccgggag   1620
   gtactcagaa aacatttctc ggcagaccag tgagaccgca gataacgaca atgcgtcgtc   1680
   cttcactatg tgatagaaag ctgagtctcc ctaaggcatg tgtgaaacat actcatagat   1740
   gttatgcaaa aaaaagtcta tggccaggta tgcatggaaa atgtgggaat taagcaaaat   1800
   caagcaagcc tctctcctgc gggacttaac gtgctcatgg gctgtgtgat ctcttcaggg   1860
   tggggtggtc tctgataggt agcattttcc agcactttgc aaggaatgtt ttgtagctct   1920
   agggtatata tccgcctggc atttcacaaa acagcctttg ggaaatgctg aattaaagtg   1980
   aattgttgac aaatgtaaac attttcagaa atattcatga agcggtcaca gatcacagtg   2040
   tcttttggtt acagcacaaa atgatggcag tggtttgaaa aactaaaaca gaaaaaaaaa   2100
   tggaagccaa cacatcactc attttaggca aatgtttaaa catttttatc tatcagaatg   2160
   tttattgttg ctggttataa gcagcaggat tggccggcta gtgtttcctc tcatttccct   2220
   ttgatacagt caacaagcct gaccctgtaa aatggaggtg gaaagacaag ctcaagtgtt   2280
   cacaacctgg aagtgcttcg ggaagaaggg gacaatggca gaacaggtgt tggtgacaat   2340
   tgtcaccaat tggataaagc agctcaggtt gtagtgggcc attaggaaac tgtcggtttg   2400
   ctttgatttc cctgggagct gttctctgtc gtgagtgtct cttgtctaaa cgtccattaa   2460
   gctgagagtg ctatgaagac aggatctaga ataatcttgc tcacagctgt gctctgagtg   2520
   cctagcggag ttccagcaaa caaaatggac tcaagagaga tttgattaat gaatcgtaat   2580
   gaagttgggg tttattgtac agtttaaaat gttagatgtt tttaattttt taaataaatg   2640
   gaatactttt tttttttttt taaagaaagc aactttactg agacaatgta gaaagaagtt   2700
   ttgttccgtt tctttaatgt ggttgaagag caatgtgtgg ctgaagactt ttgttatgag   2760
   gagctgcaga ttagctaggg gacagctgga attatgctgg cttctgataa ttattttaaa   2820
   ggggtctgaa atttgtgatg gaatcagatt ttaacagctc tcttcaatga catagaaagt   2880
   tcatggaact catgttttta aagggctatg taaatatatg aacattagaa aaatagcaac   2940
   ttgtgttaca aaaatacaaa cacatgttag gaaggtactg tcatgggcta ggcatggtgg   3000
   ctcacacctg taatcccagc attttgggaa gctaagatgg gtggatcact tgaggtcagg   3060
   agtttgagac cagcctggcc aacatggcga aacccctctc tactaaaaat acaaaaattt   3120
   gccaggcgtg gtggcgggtg cctgtaatcc cagctacttg ggaggctgag gcaagagaat   3180
   cgcttgaacc caggaggcag aggttgcagt gagccgagat cgtgccattg cactccagcc   3240
   tgggtgacaa agcgagactc catctcaaaa aaaaaaaaaa aaaaaaagga aagaactgtc   3300
   atgtaaacat accaacatgt ttaaacctga caatggtgtt atttgaaact ttatattgtt   3360
   cttgtaagct ttaactatat ctctctttaa aatgcaaaat aatgtcttaa gattcaaagt 3420
   ctgtattttt aaagcatggc tttggctttg caaaataaaa aatgtgtttt gtacatgaa 3479
<210> 9
   <211> 793
   <212> DNA
   <213> Homo sapiens
<400> 9
   aggagttgtg agtttccaag ccccagctca ctctgaccac ttctctgcct gcccagcatc 60
   atgaagggcc ttgcagctgc cctccttgtc ctcgtctgca ccatggccct ctgctcctgt 120
   gcacaagttg gtaccaacaa agagctctgc tgcctcgtct atacctcctg gcagattcca 180
   caaaagttca tagttgacta ttctgaaacc agcccccagt gccccaagcc aggtgtcatc 240
   ctcctaacca agagaggccg gcagatctgt gctgacccca ataagaagtg ggtccagaaa 300
   tacatcagcg acctgaagct gaatgcctga ggggcctgga agctgcgagg gcccagtgaa 360
   cttggtgggc ccaggaggga acaggagcct gagccagggc aatggccctg ccaccctgga 420
   ggccacctct tctaagagtc ccatctgcta tgcccagcca cattaactaa ctttaatctt 480
   agtttatgca tcatatttca ttttgaaatt gatttctatt gttgagctgc attatgaaat 540
   tagtattttc tctgacatct catgacattg tctttatcat cctttcccct ttcccttcaa 600
   ctcttcgtac attcaatgca tggatcaatc agtgtgatta gctttctcag cagacattgt 660
   gccatatgta tcaaatgaca aatctttatt gaatggtttt gctcagcacc accttttaat 720
   atattggcag tacttattat ataaaaggta aaccagcatt ctcactgtga aaaaaaaaaa 780
   aaaaaaaaaa aaa 793
<210> 10
   <211> 851
   <212> DNA
   <213> Homo sapiens
<400> 10
   agaatataac agcactccca aagaactggg tactcaacac tgagcagatc tgttctttga      60
   gctaaaaacc atgtgctgta ccaagagttt gctcctggct gctttgatgt cagtgctgct     120
   actccacctc tgcggcgaat cagaagcagc aagcaacttt gactgctgtc ttggatacac     180
   agaccgtatt cttcatccta aatttattgt gggcttcaca cggcagctgg ccaatgaagg     240
   ctgtgacatc aatgctatca tctttcacac aaagaaaaag ttgtctgtgt gcgcaaatcc     300
   aaaacagact tgggtgaaat atattgtgcg tctcctcagt aaaaaagtca agaacatgta     360
   aaaactgtgg cttttctgga atggaattgg acatagccca agaacagaaa gaaccttgct     420
   ggggttggag gtttcacttg cacatcatgg agggtttagt gcttatctaa tttgtgcctc     480
   actggacttg tccaattaat gaagttgatt catattgcat catagtttgc tttgtttaag     540
   catcacatta aagttaaact gtattttatg ttatttatag ctgtaggttt tctgtgttta     600
   gctatttaat actaattttc cataagctat tttggtttag tgcaaagtat aaaattatat     660
   ttggggggga ataagattat atggactttc ttgcaagcaa caagctattt tttaaaaaaa     720
   actatttaac attcttttgt ttatattgtt ttgtctccta aattgttgta attgcattat     780
   aaaataagaa aaatattaat aagacaaata ttgaaaataa agaaacaaaa agttcttctg     840
   ttaaaaaaaa a                                                          851
<210> 11
   <211> 848
   <212> DNA
   <213> Homo sapiens
<400> 11
   agaatataac agcactccca aagaactggg tactcaacac tgagcagatc tgttctttga      60
   gctaaaaacc atgtgctgta ccaagagttt gctcctggct gctttgatgt cagtgctgct     120
   actccacctc tgcggcgaat cagaagcaag caactttgac tgctgtcttg gatacacaga     180
   ccgtattctt catcctaaat ttattgtggg cttcacacgg cagctggcca atgaaggctg     240
   tgacatcaat gctatcatct ttcacacaaa gaaaaagttg tctgtgtgcg caaatccaaa     300
   acagacttgg gtgaaatata ttgtgcgtct cctcagtaaa aaagtcaaga acatgtaaaa     360
   actgtggctt ttctggaatg gaattggaca tagcccaaga acagaaagaa ccttgctggg     420
   gttggaggtt tcacttgcac atcatggagg gtttagtgct tatctaattt gtgcctcact     480
   ggacttgtcc aattaatgaa gttgattcat attgcatcat agtttgcttt gtttaagcat     540
   cacattaaag ttaaactgta ttttatgtta tttatagctg taggttttct gtgtttagct     600
   atttaatact aattttccat aagctatttt ggtttagtgc aaagtataaa attatatttg     660
   ggggggaata agattatatg gactttcttg caagcaacaa gctatttttt aaaaaaaact     720
   atttaacatt cttttgttta tattgttttg tctcctaaat tgttgtaatt gcattataaa     780
   ataagaaaaa tattaataag acaaatattg aaaataaaga aacaaaaagt tcttctgtta     840
   aaaaaaaa                                                              848
<210> 12
   <211> 20
   <212> DNA
   <213> Artificial
<220>
   <223> forward primer GAPDH
<400> 12
   caccagggct gcttttaact 20
<210> 13
   <211> 20
   <212> DNA
   <213> Artificial
<220>
   <223> reverse primer GAPDH
<400> 13
   gatctcgctc ctggaagatg 20
<210> 14
   <211> 20
   <212> DNA
   <213> Artificial
<220>
   <223> forward primer CCR6
<400> 14
   gcacaaaatg atggcagtgg 20
<210> 15
   <211> 20
   <212> DNA
   <213> Artificial
<220>
   <223> reverse primer CCR6
<400> 15
   ccgaagcact tccaggttgt 20
<210> 16
   <211> 22
   <212> DNA
   <213> Artificial
<220>
   <223> forward primer collagen type I
<400> 16
   ccctgtctgc ttcctgtaaa ct 22
<210> 17
   <211> 22
   <212> DNA
   <213> Artificial
<220>
   <223> reverse primer collagen type I
<400> 17
   catgttcggt tggtcaaaga ta 22
<210> 18
   <211> 20
   <212> DNA
   <213> Artificial
<220>
   <223> forward primer alphaSMA
<400> 18
   catcatgcgt ctggatctgg 20
<210> 19
   <211> 20
   <212> DNA
   <213> Artificial
<220>
   <223> reverse primer alphaSMA
<400> 19
   ggacaatctc acgctcagca 20

## Description

### FIELD OF THE INVENTION

The present invention relates to pharmaceutical compositions comprising inhibitors of CCR6 receptor activity and/or expression.

### BACKGROUND OF THE INVENTION

Interstitial lung diseases are a heterogenous group of disorders accompanied by various degrees of in flammation and fibrosis resulting in the damage of the lung parenchyma. Recently, the subgroup of idiopathic interstitial pneumonias has been classified into seven different syndromes. The most frequent of these conditions arc idiopathic pulmonary fibrosis (IPF), non-specific interstitial pneumonia (NSIP) and cryptogenic organizing pneumonia (COP).

The etiology of said diseases has remained elusive and the molecular mechanisms driving their pathogenesis arc poorly understood. However, the final pathway of fibroblast proliferation and extracellular matrix release is analyzed in detail and represents a common pathway of fibrosing lung diseases of known and unknown etiology including collagen-vascular and systemic inflammatory diseases with pulmonary manifestations leading to fibrosis (e.g. rheumatoid arthritis, systemic sclerosis, scleroderma, hypersensitivity pneumonitis, some forms of drug induced alveolitis).

IPF is a distinctive type of chronic fibrosing interstitial pneumonia of unknown cause. When lung tissue from patients suffering from IPF is examined, it shows a characteristic set of histologic/pathologic features known as usual interstitial pneumonia (UIP). NSIP in contrast refers to cases of interstitial pneumonia in which a distinct pattern of more homogenous inflammation and fibrosis different from UIP can be identified.

IPF is slightly more common in males than in females and usually occurs in patients of 50 years of age or older.

The most common symptoms of IPF are shortness of breath, especially during or after physical activity, and a dry cough. Said symptoms often don't appear until the disease is advanced, and irreversible lung damage has already occurred.

The prognosis of IPF is rather poor with an average survival time of 3 years from the time of diagnosis.

At present no effective treatments and no cure for pulmonary fibrosis are available. Often treatment is limited to treating the inflammatory response that occurs in the lungs.

Standard therapy includes anti-inflammatory and cytotoxic drugs like steroids and cyclophosphamide or azathioprine, however, these therapies are only of some value in e.g. NSIP (which also has a better prognosis than IPF) or desquamative interstitial pneumonia (DIP). IPF, however, is refractory to most of these therapeutic options. Experimental therapy studies using IFNγ, Bosentan® (endothelin antagonist), Aviptadil® (vasoactive intestinal peptide, VIP) or the tyrosine kinase inhibitor Imatinib® did also not reveal any profound beneficial effect of these drugs.

The development of novel therapies for the treatment of the various forms of pulmonary fibrosis, especially of IPF, therefore remains an essential task. There is a need for new cellular targets as well as therapeutic molecules which can efficiently impinge on these targets.

Chemokines are a family of proinflammatory cytokines which are essential for homeostasis and activation of the immune system. They bind to specific cell surface receptors belonging to the family of seven-transmembrane domain, G protein-coupled receptors.

CCL18, also known as pulmonary and activation-regulated chemokine (PARC), alternative macrophage activation-associated CC chemokine I (AMAC- 1), macrophage inflammatory protein-4 (MIP-4) and dendritic cell-derived chemokine1 (DCCK1), is a chemokine that is mainly expressed by a broad range of moncytes/macrophages and dendritic cells. It is constitutively expressed at high levels in human lung. CCL18 attracts T cells, immature dendritic cells, and induces collagen synthesis by fibroblasts. Furthermore, there arc hints that CCL18 might also induce the chcmotaxis of B cells.

CCL18 levels are enhanced in various disease states, such as e.g. inflammatory disorders of the skin, lung and joints, It has also been found that CCL18 is released in elevated levels by alveolar macrophages from patients suffering from pulmonary fibrosis and the serum levels of this chemokine is a prognostic marker in fibrotic diseases.

Moreover, it could be demonstrated that CCL18 induces the differentiation of fibroblasts into myo-fibroblasts and induces the expression of collagen and α-smooth-muscle-actin. Due to its known collagen inducing properties, high levels of CCL18 might directly be linked to the increased matrix deposition in pulmonary fibrosis.

However, exact analysis of signaling events and possible therapeutic interventions in CCL18 signaling is hampered by the fact that its receptor is not know.

Cancer is a class of diseases in which a group of cells display uncontrolled growth, invasion and sometimes metastasis. These three malignant properties of cancer differentiate them from benign tumors, which are self-limited and do not invade or metastasize.

Cancer is a major health problem causing about 13% of all deaths worldwide. According to the American Cancer Society, 7.6 million people died from cancer in the world during 2007. Deaths from cancer arc projected to continue rinsing, with an estimated 12 million deaths in 2030.

### The development of novel therapies to combat cancer therefore remains an essential task.

WO 01/32874 entitled "Novel polypeptides and nucleic acids encoding same" is directed to isolated NOVX polynucleotides and polypeptides encoded by the NOVX polynucleotides. WO 01/032874 also provides antibodies that immunospecifically bind to a NOVX polypeptide or any derivative, variant, mutant or fragment of the NOVX polypeptide, polynucleotide or antibody. Finally, WO 01/32874 provides methods in which the NOVX polypeptide, polynucleotide and antibody are utilized in the detection and treatment of a broad range of pathological states.

EP 1 422 239 entitled "Mobilization of hematopoietic stem cells using a chemokine" is directed to novel chemokines for mobilizing stem cells and methods of mobilizing stem cells.

WO 00/55180 entitled "human lung cancer associated gene sequences and polypeptide" relates to newly identified lung or lung cancer related polynucleotides and the polypeptides encoded by these polynucleotides, and to the complete gene sequences associated therewith and to the expression products thereof. WO 00/55180 *inter alia* also relates to the use of such polynucleotides and polypeptides for detection, prevention and treatment of disorders of the lung, particularly the presence of lung cancer. Therapeutic methods of treating such disorders are also disclosed in WO 00/55180.

### Objective and summary of the invention

It is one objective of the present invention to provide a pharmaceutical composition comprising an inhibitor of CCR6 receptor activity.

Another object of the present invention is to provide pharmaceutical compositions comprising compounds suitable for the treatment of an interstitial lung disease and/or cancer, wherein said cancer is preferably an adenocarcinoma, most preferable an adenocarcinoma of the lung.

In a first aspect the present invention provides a pharmaceutical composition according to claim 1.

In another aspect the present invention relates to a pharmaceutical composition according to claim 1 for use in the treatment of an interstitial lung disease and/or cancer.

### FIGURE LEGENDS:

**Figure 1** CCR6 mRNA expression by different fibroblast lines from patients suffering from squamous carcinoma (n=3), NSIP (n=2) and UIP (n=3). CCR6 expression was normalized using the housekeeping gene Glycerinaldehyd-3-phosphat-dehydrogenase (GAPdH).
**Figure 2** Analysis of CCR6 expression by different fibroblast lines from patients suffering from UIP (upper panel, left and mid), NSIP (upper panel right), and squamous carcinoma (SQ CA; lower panel).
**Figure 3** CCR6 is not expressed in control lung (A), however, in fibrotic lungs expression of CCR6 can be found at the apical surface of alveolar epithelial cells (B and C, arrow heads) and on fibroblasts (C, arrows) (magnification: A: x100, B: x200, C; x400).
**Figure 4** Unstimulated (unst.) FGF2 release is increased in fibroblasts from fibrotic lungs (grey, n=6 (UIP n=3, sarcoidosis n=1, NSIP n=1, undefined n=1)) compared with fibroblasts from non-fibrotic lungs (light grey, "Contr.", n=6). CCL18 induces a significant up-regulation of FGF2 release in fibroblasts from fibrotic lungs but only marginal in fibroblasts from non-fibrotic lungs. Blockade of CCR6 with a blocking antibody inhibits the CCL18 induced up-regulation of FGF2 release. Again, this effect is only seen in fibroblasts from fibrotic lungs.
**Figure 5** CCL18 induced collagen I mRNA expression in three out of four (upper panel) and alpha-smooth muscle actin (αSMA) mRNA expression in all investigated human lung fibroblast cell lines (lower panel) (ordinate indicates line names). Collagen I mRNA expression and alpha-smooth muscle actin (αSMA) mRNA expression is blocked by anti- CCR6 (rE=relative Expression).
**Figure 6** The transformed rat alveolar epithelial cell line RLE-6TN undergoes epithelial-mesenchymal transition (EMT) after stimulation with TGFβ or CCL18. Arrows indicate fibroblast like cells. Culture period = 6 days.
**Figure 7** Western Blot analysis of the expression of vimentin and αSMA cultured for 6 days either un-stimulated or in the presence of TGFβ, TGFβ+TNFα, CCL18, or CCL18+TNFα.
**Figure 8** Immuno-reactivity for αSMA was assessed by immuno-fluorescence on day 6. RLE-6TN cells were left unstimulated (A) or stimulated with (B) TGFβ+TNFα, (C) CCL18 (D), or CCL18+TNFα. Nuclei arc strained by DAPI. αSMA is only visible in panel B uncovering the typical shape of fibroblasts (arrow heads). In contrast, in panel C and D only nuclei are evident.
**Figure 9** Immuno-reactivity of CD90 was assessed by immuno-fluorescence on day 6. RLE-6TN cells were left unstimulated (A) or stimulated with TGFβ+TNFα (B), CCL18 (C). Nuclei arc stained by ToPro3. Unstimulated cells do not express CD90 and only the nuclei arc visible. In contrast, both TGFβ+TNFα and CCL18 stimulated cells express CD90 as demonstrated by the uncovering of the cell shape.
**Figure 10** CCL18 induced expression of αSMA is found in CCR6 transfected RLE-6TN but not in mock-transfected cells. In contrast, TGFβ induce αSMA expression in both cell lines.
**Figure 11** human primary AECII were stimulated 12 days with TGFβ+TNFα or CCL18 in absence or presence of TNFα. Total cell lysate was separated by 10% SDS-PAGE and analyzed by Western blot. Non-stimulated cells only marginally express α-smooth muscle actin (αSMA) and no vimentin. Stimulation with TGFβ+TNFα and CCL18 alone or in combination with TNFα strongly up-regulates both molecules. Interestingly, cytokeratin is down-regulated by TGFβ+TNFα but preserved by CCL18 alone or in combination with TNFα.
**Figure 12** Expression of CCR6 by peripheral blood mononuclear cells after 7 days of culture in the presence or absence of phytohemagglutinin (PHA; 5µg/ml). Cell preparations were grouped in "high" or "low" CCR6 expressing preparations according to their initial CCR6 expression. After the culture period, non-stimulated preparations did not charge CCR6 expression pattern. In contrast, stimulation with PHA reduced CCR6 expression in the high expression group but increased expression in the low expression group.
**Figure 13** Down-regulation of CCR6 receptor after 20 minutes incubation with CCL18 (10 ng/ml). Incubation with CCL18 leads to a marked down-regulation of the CCR6 surface expression. This effect might be caused by receptor internalization.

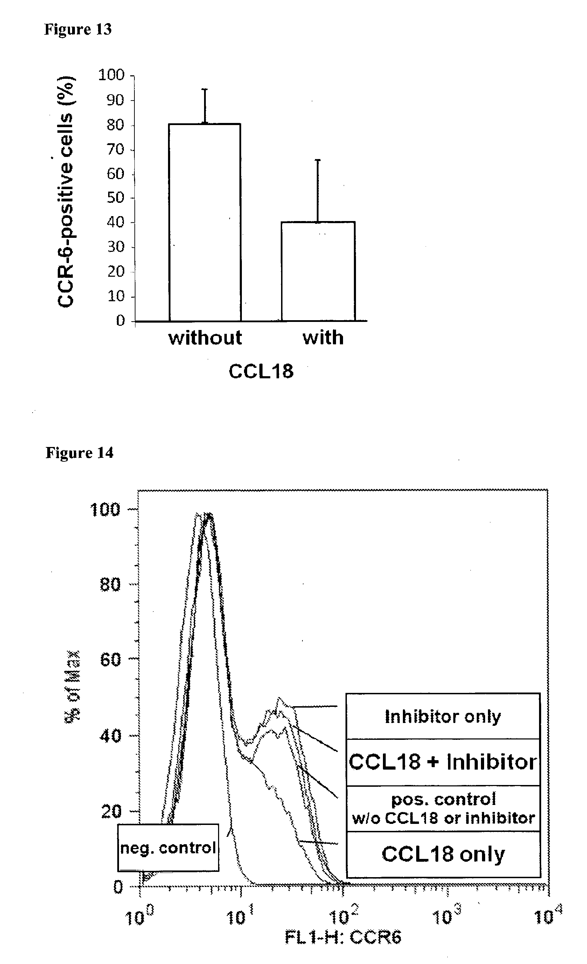

**Figure 14** FACS analysis of CCL18 induced down-regulation of CCR6 expression on human lymphocytes and its inhibition by the inhibitor PS-AU-1015 (polypeptide according to SEQ ID NO:.1). A subpopulation of freshly isolated human lymphocytes express the chemokine receptor CCR6 visible as a lower peak right hand side of the main peak (pos. control). Incubation with CCL18 (10ng/ml) for 20 minutes diminishes the peak markedly (CCL18 only). Upon incubation of the cells with CCL18 (10ng/ml) in the presence of the inhibitor PS-AU-1015 (SEQ ID NO:.1; 100ng/ml) this down-regulation docs not occur (CCL18 + inhibitor). Inhibitor only exhibits no effect.
**Figure 15** CCR6 staining in lung sections from control lung (A) and two adenocarcinoma lungs (B, C). No staining is visible in the control lung whereas tumour cells are positive for CCR6 (red colour, arrows).
**Figure 16** Expression of CCR6 on the surface of lung adenocarcinoma cells (upper panel) and pleural mesothelioma cells (lower panel). Left peak indicates isotype control, right peak indicates CCR6 expression. Two out of three adenocarcinoma cell lines and all pleural mesothelioma cell lines exhibit marked CCR6 expression.
**Figure 17** Listing of primers used for PCR.

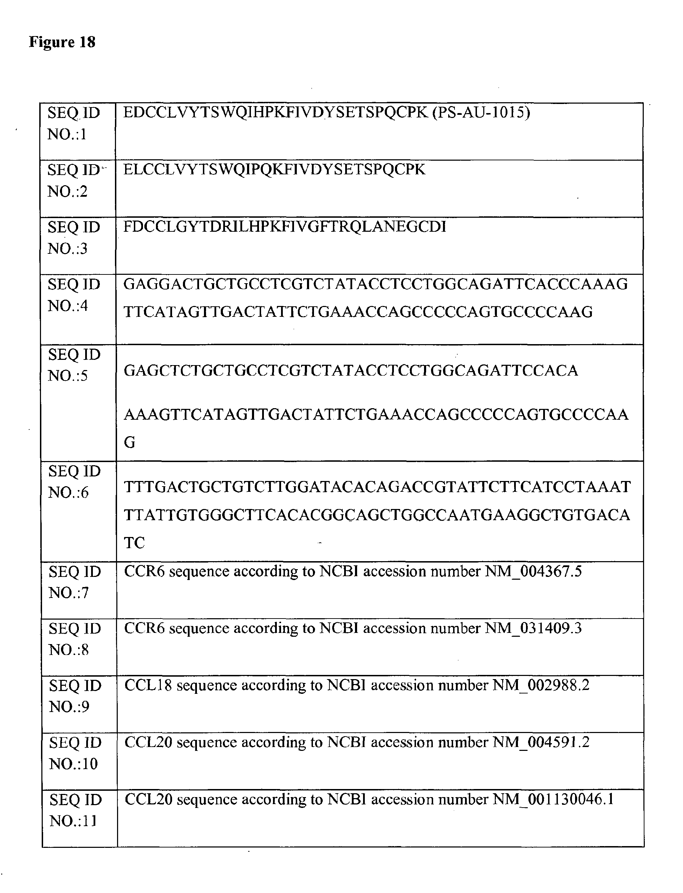

**Figure 18** Sequence listing SEQ ID NO: 1 to 11.

### DETAILED DESCRIPTION OF THE INVENTION

Unless defined otherwise, all technical and scientific terms used herein have the same meanings as commonly understood by one of ordinary skill in the art.

The following definitions arc introduced.

As used in this specification and in the intended claims, the singular forms of "a" and "an" also include the respective plurals unless the context clearly dictates otherwise.

It is to be understood that the term "comprise", and variations such as "comprises" and "comprising" is not limiting. For the purpose of the present invention the term "consisting of" is considered to be a preferred embodiment of the term "comprising". If hereinafter a group is defined to comprise at least a certain number of embodiments, this is meant to also encompass a group which preferably consists of these embodiments only.

The terms "about" and "approximately" in the context of the present invention denote an interval of accuracy that a person skilled in the art will understand to still ensure the technical effect of the feature in question. The term typically encompasses a deviation from the indicated numerical value of ±10% and preferably of ±5 %.

The determination of percent identity between two sequences is preferably accomplished using the mathematical algorithm of Karlin and Altschul (1993) Proc. Natl. Acad. Sci USA 90: 5873-5877. Such an algorithm is e.g. incorporated into the BLASTn and BLASTp programs of Altschul ct at. (1990) J. Mol. Biol. 215: 403-410 available at NCBI (http://www.ncbi.nlm.nih.gov/blast/Blast.cgi).

The determination of percent identity is preferably performed with the standard parameters of the BLASTn and BLASTp programs.

BLAST polynucleotide searches arc preferably performed with the BLASTn program.
For the general parameters, the "Max Target Sequences" box may be set to 100, the "Short queries" box may be ticked, the "Expect threshold" box may be set to 10 and the "Word Size" box may be set to 28. For the scoring parameters the "Match/mismatch Scores" may be set to 1,-2 and the "Gap Costs" box may be set to linear. For the Filters and Masking parameters, the "Low complexity regions" box may not be ticked, the "Species-specific repeats" box may not be ticked, the "Mask for lookup table only" box may be ticked, the "Mask lower case letters" box may not be ticked.

BLAST protein searches arc preferably performed with the BLASTp program.
For the general parameters, the "Max Target Sequences" box may be set to 100, the "Short queries" box may be ticked, the "Expect threshold" box may be set to 10 and the "Word Size" box may be set to "3".

For the scoring parameters the "Matrix" box may be set to "BLOSUM62", the "Gap Costs" Box may be set to "Existence: 11 Extension: 1", the "Compositional adjustments" box may be set to "Conditional compositional score matrix adjustment". For the Filters and Masking parameters the "Low complexity regions" box may not be ticked, the "Mask for lookup table only" box may not be ticked and the "Mask lower case letters" box may not be ticked.

The percent identity is determined over the entire length of the respective reference sequence, i.e. over the entire length of the sequence according to the SEQ ID NO or SEQ ID NOs recited in the respective context. For example, an amino acid sequence which exhibits at least 80% identity to the sequence according to SEQ ID NO.:1 exhibits at least 80% identity to SEQ ID NO.:1 over the entire length of SEQ ID NO.:1. In another example, a sequence which exhibits at least 80% identity to the sequence according to SEQ ID NO.:4 exhibits at least 80% identity to SEQ ID NO.:4 over the entire length of SEQ ID NO.:4.

The term "subject" as used herein refers to a human or an animal, preferably a mammal such as e.g. non-human primates, mice, rats, rabbits, guinea pigs, dogs, cats, castle, horses, sheep, pigs, goats and the like. Preferably a "subject" in the context of the present invention is a human.

The terms "chemokine receptor CCR6" or "CCR6 receptor", "Chemokine ligand 18" or "CCL18" and "Chemokine ligand 20" or "CCL20" mentioned in the context of the present invention are well known in the art. Therefore the average skilled person can easily retrieve the polynucleotide and amino acid sequences of CCR6 receptor, CCL18 and CCL20 and orthologous and splice isoforms thereof from any suitable public database such as e.g. the NCB1 database (http://www.ncbi.nlm.nih.gov/pubmed/). The terms "CCR6 receptor" and "CCR6" are used interchangeably herein.

"CCR6 receptor", "CCL18" and "CCL20" as mentioned in the context of the present invention are preferably mammalian CCR6 receptor, CCL18 and CCL20, most preferably human CCR6 receptor and human CCL18 or human CCL20. Human CCR6 receptor can e.g. be found in the NCBI database under accession number NM_004367.5 (transcript variant 1; SEQ ID NO.:7) or NM_031409.3 (transcript variant 2; SEQ ID NO.:8). Human CCL18 can e.g. be found in the NCBI database under accession number NM_002988.2 (SEQ ID NO.:9). Human CCL20 can e.g. be found in the NCBI database under accession number MM_004591.2 (transcript variant 1; SEQ ID NO.:10) or NM_001130046.1 (transcript variant 2; SEQ ID NO.:11).

CCR6 is sometimes also referred to as CD196. CCL18 is sometimes also referred to as pulmonary and activation-regulated chemokine (PARC), alternative macrophage activation-associated CC chemokine 1 (AMAC-1), macrophage inflammatory protein-4 (MIP-4) or dendritic cell-derived chemokine 1 (DCCK1).

The terms "IPF" (idiopathic pulmonary fibrosis) and "UIP" (usual interstitial pneumonia) are used synonymously herein.

The inventors of the present invention surprisingly found that CCL18 is an agonist of CCR6 receptor, a member of the seven-transmembrane G-protein-coupled chemokine receptor family. The inventors further surprisingly found that inhibitors of CCR6 receptor activity can be used for the therapy of an interstitial lung disease or cancer.

Therefore in one aspect the present invention relates to a pharmaceutical composition as claimed in claim 1.

Preferably said CCR6 receptor is human CCR6 receptor. Most preferably said CCR6 receptor is a polypeptide according to SEQ ID NO.: 7 or SEQ ID NO.: 8.

The term "isolated" in the context of the present invention indicates that a polypeptide or polynucleotide has been removed from its natural environment and/or is presented in a form in which it is not found in nature. An "isolated" polypeptide or an "isolated" polynucleotide may also be a polypeptide or polynucleotide that has been generated in vitro.

In a preferred example the amino acid sequence according to (a) exhibits at least 91%, even more preferably at least 92%, even more preferably at least 93%, even more preferably at least 94%, even more preferably at least 95%, even more preferably at least 96%, even more preferably at least 97%, even more preferably at least 98% or even more preferably at least 99% identity to the sequence according to EQ ID NO.:1. In a most preferred example the amino acid sequence according to (a) exhibits 100% identity to the sequence according to SEQ ID NO.:1.

In some preferred examples the amino acid sequence according to (a) may exhibit at least 80%, preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98% or even more preferably at least 99% identity to the sequence according to SEQ ID NO.: 1.

In another preferred examples in the amino acid sequence according to (a) not more than 1, 2, 3, 4, 5, 6, 7, 8, 9 or 10 amino acids of the sequence according to SEQ ID NO:1 are changed (i.e. deleted, inserted, modified and/or substituted by other amino acids).

A substitution in an amino acid sequence may be a conservative or a non-conservative substitution, preferably a conservative substitution. A substitution also includes the exchange of a naturally occurring amino acid with a non-natural amino acid. A conservative substitution comprises the substitution of an amino acid with another amino acid having a chemical property similar to the amino acid that is substituted. Preferably, the conservative substitution is a substitution selected from the group consisting of:
(i) a substitution of a basic amino acid with another, different basic amino acid;
(ii) a substitution of an acidic amino acid with another, different acidic amino acid;
(iii) a substitution of an aromatic amino acid with another, different aromatic amino acid;
(iv) a substitution of a non-polar, aliphatic amino acid with another, different non-polar, aliphatic amino acid; and
(v) a substitution of a polar, uncharged amino acid with another, different polar, uncharged amino acid.

A basic amino acid is preferably selected from the group consisting of arginine, histidine, and lysine. An acidic amino acid is preferably aspartate or glutamate.

An aromatic amino acid is preferably selected from the group consisting of phenylalanine, tyrosinc and tryptophane. A non-polar, aliphatic amino acid is preferably selected from the group consisting of glycine, alanine, valine, leucine, methionine and isoleucine. A polar, uncharged amino acid is preferably selected from the group consisting of serine, threonine, cystcinc, proline, asparagine and glutamine. In contrast to a conservative amino acid substitution, a non-conservative amino acid substitution is the exchange of an amino acid with any amino acid that does not fall under the above-outlined conservative substitutions (i) through (v).

Amino acids of the isolated polypeptide may also be modified, e.g. chemically modified. For example, the side chain or a free amino or carboxy-terminus of an amino acid of the protein may be modified by e.g. glycosylation, amidation, phosphorylation, ubiquitination, etc.

In a preferred embodiment the amino acid sequence according to (a) comprises at least 8 consecutive amino acids of the sequence according to SEQ ID NO.: 1. Thus, the amino acid sequence according to (a) exhibits at least 80%, preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98% , even more preferably at least 99% or most preferably 100% identity to the sequence according to SEQ ID NO.:1 and comprises at least 8 consecutive amino acids of the sequence according to SEQ ID NO.:1. In another preferred example the amino acid sequence according to (a) exhibits at least 80%, preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferable at least 98% , even more preferably at least 99% or most preferably 100% identity to the sequence according to SEQ ID NO.: 1 and comprises at least 8 or at least 15 consecutive amino acids of the sequence according to SEQ ID NO.:1. In another preferred example the amino acid sequence according to (a) exhibits at least 95%, 98% or 100% identity to the sequence according to SEQ ID NO.:1 and comprises at least 9, at least 10, at least 11, at least 1 2, at least 13, at least 14, at least 15, at least 16, at least 17, at least 18, at least 19 or at least 20 consecutive amino acids of the sequence according to SEQ ID NO.:1. In a more preferred example, the amino acid sequence according to (a) exhibits at least 95%, 98% or 100% identity to the sequence according to SEQ ID NO.:1 and comprises at least 8 or at least 15 consecutive amino acids of the sequence according to SEQ ID NO.:1.

A fragment is typically a portion of the amino acid sequence it refers to.

In a preferred example the fragment according to (b) is at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27 or at least 28 amino acids in length. In a preferred embodiment the fragment according to (b) has a length of at least 10, at least 15 or at least or at least 20 amino acids. In a most preferred example the fragment according to (b) has a length of 15 amino acids.

In another particularly preferred embodiment the isolated polypeptide according to 1) consists of an amino acid sequence according to SEQ ID NO.: 1, wherein said polypeptide is capable of binding to and inhibiting the activity of the CCR6 receptor.

Whether a polypeptide according to the invention is capable of binding to the CCR6 receptor may be determined by any suitable method known to the skilled person. For example, the skilled person my determine whether a polypeptide is capable of binding to the CCR6 receptor by using a yeast two-hybrid assay or a biochemical assay such as e.g. a pull-down assay, a co-immunoprecipitation assay, an enzyme-linked immunosorbent assay (ELISA), a quantitative radioligand binding assay, a fluorescence-activated cell sorting (FACS)-based assay, a Plasmon resonance assay or any other method known to the skilled person. When using pull-down or Plasmon resonance assays, it is useful to fuse at least one of the proteins to an affinity tag such as HIS-tag, GST-tag or other, as is well known in the art of biochemistry.

The term "inhibiting the activity of the CCR6 receptor" as used herein means that CCR6 receptor activity is downregulated or abolished and/or that the activation of CCR6 receptor is inhibited.

For example, an isolated polypeptide or any other compound capable of inhibiting the activity and/or the expression of the CCR6 receptor described herein may sterically block the CCR6 receptor, such that a CCR6 receptor agonist (e.g. CCL18 or CCL20) cannot activate the receptor. Thus, an inhibition of CCR6 receptor activity may e.g. be due to an inhibition of the interaction of the CCR6 receptor with one of its ligands, i.e. CCL18 and/or CCL20, by a polypeptide or any other compound that binds to the CCR6 receptor but does not mediate a signaling event by itself.

An isolated polypeptide according to 1) capable of inhibiting the activity and/or the expression of the CCR6 receptor may down regulate or abolish existing CCR6 receptor activity.

Whether an isolated polypeptide is capable of inhibiting the activity of the CCR6 receptor may e.g. be determined by measuring cell surface expression of CCR6 as described herein below in example 6 and figures 13 and 14, wherein an inhibition of CCR6 receptor internalisation in presence of a CCR6 receptor agonist (such as e.g. CCL18) and the isolated polypeptide, in comparison to a control (e.g. presence of CCR6 receptor agonist only) indicates that the isolated polypeptide is capable of inhibiting the activity of the CCR6 receptor.

In another example, the skilled person may also determine the ability of an isolated polypeptide according to 1) to inhibit CCR6 receptor activity by incubating CCR6 expressing cells in the presence of a CCR6 receptor agonist, such as e.g. CCL18 or CCL20, and in the presence or absence of the isolated polypeptide and subsequently lysing the cells and analysing phosphorylation of ERK, a downstream molecule of CCR6 signaling, by Western blot analysis. In this example a lower level of phosphorylation of ERK in the presence of the polypeptide versus the level of phosphorylation of ERK in the absence of the isolated polypeptide indicates that the polypeptide is capable of inhibiting the activity of the CCR6 receptor. One method for determining ERK phosphorylation by Western Blot analysis is e.g. described in Lin et al. J Proteome Res. 2010 Jan;9(1):283-97. In an analogous example, the phosphorylation of other CCR6 downstream effectors such as e.g. Akt, SAPK/JNK kinases, phosphatidylinositol 3-kinase or phospholipase C may be analysed in order to determine whether an isolated polypeptide according to the invention is capable of inhibiting CCR6 receptor activity.

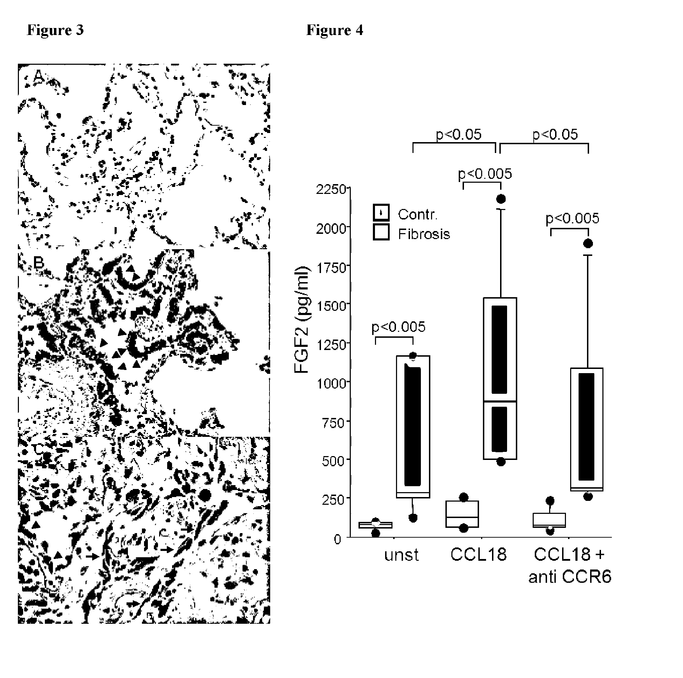

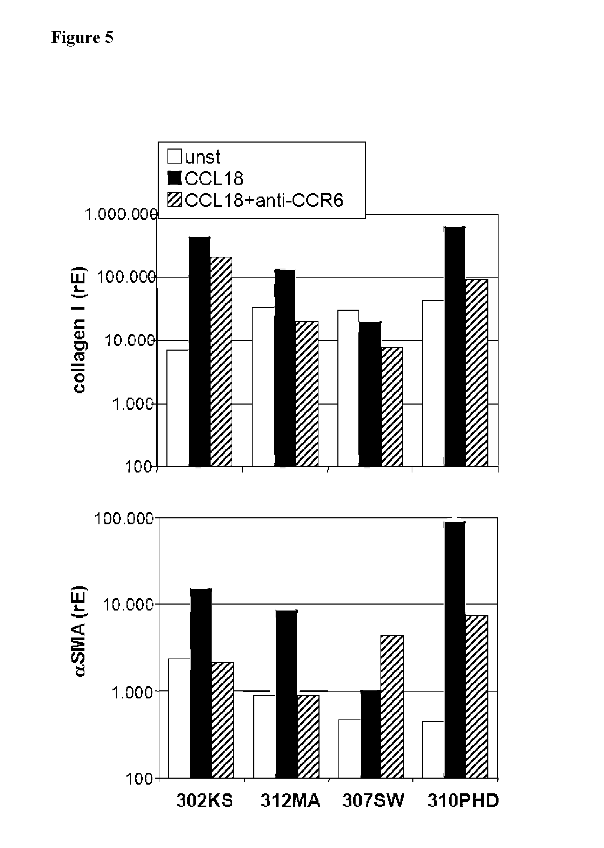

Whether an isolated polypeptide according to 1) is capable of inhibiting the activity of the CCR6 receptor may e.g. also be determined by measuring CCL18 stimulated FGF2 release or CCL1A mediated induction of collagen and/or α-SMA in the presence of said polypeptide as e.g. described herein below in example 4 and figures 4 and 5. In this example an inhibition of CCL18 induced FGF2 up regulation and/or an inhibition of the CCL18 mediated induction of collagen and/or α-SMA expression in the presence of the polypeptide in comparison to a control, to which the polypeptide has not been added, indicates that the isolated polypeptide is capable of inhibiting the activity of the CCR6 receptor. Furthermore, in order to determine the ability of an isolated polypeptide according to 1) to inhibit the activity of the CCR6 receptor, the skilled person may also determine whether the induction of Epithelial-Mesenchymal-Transition (EMT) by CCL18 is down regulated or abolished.

Alternatively or additionally any further methods which arc suited to determine the activitiy of the CCR6 receptor and which arc comprised in the art and known to the average skilled person may be used.

An isolated polypeptide according to 1) which is capable of inhibiting the activity of the CCR6 receptor, may inhibit the activity of the CCR6 receptor by at least 10%, at least 20%, at least 30%, at least 40%, at least 50%, at least 60%, at least 70%, more preferably at least 80%, at least 90%, at least 91%, at least 92%, at least 93%, at least 94%, at least 95%, at least 96%, at least 97%, at least 98% or at least 99% when compared to a control. A polypeptide according to 1), which is capable of inhibiting the activity of the CCR6 receptor, may inhibit the activity of the CCR6 receptor by 100%.

In another aspect the present invention relates to an isolated polynucleotide encoding the isolated polypeptide according to 1) or 2), wherein said nucleotide is comprised in an expression vector.

The isolated polynucleotide has a length of less than 6000 nucleotides, less than 5000 nucleotides, less than 4000 nucleotides, less than 3000 nucleotides, less than 2000 nucleotides, less than 1000 nucleotides or less than 500 nucleotides.

The isolated polynucleotide has a length of between at least 24 and 9000 nucleotides, preferably at least 24 and 8000 nucleotides, more preferably between at least 24 and 7000 nucleotides, more preferably between at least 24 and 6000 nucleotides, more preferably between at least 24 and 5000 nucleotides, more preferably between at least 24 and 4000 nucleotides, more preferably between at least 24 and 3000 nucleotides, more preferably between at least 24 and 2000 nucleotides, more preferably between at least 24 and 1000 nucleotides or even more preferably between at least 24 and 500 nucleotides. In another preferred example an isolated polynucleotide has a length of at least 24, at least 87 or at least 93 nucleotides.

The isolated polynucleotide has a length of between at least 87 and 9000 nucleotides, preferably at least 87 and 8000 nucleotides, more preferably between at least 87 and 7000 nucleotides, more preferably between at least 87 and 6000 nucleotides, more preferably between at least 87 and 5000 nucleotides, more preferably between at least 87 and 4000 nucleotides, more preferably between at least 87 and 3000 nucleotides, more preferably between at least 87 and 2000 nucleotides, more preferably between at least 87 and 1000 nucleotides or even more preferably between at least 87 and 500 nucleotides.

An isolated polynucleotide has a length of at least 87 nucleotides, at least 100 nucleotides, at least 200 nucleotides, at least 500 nucleotides, at least 800 nucleotides, at least 1000 nucleotides, at least 1500 nucleotides, at least 2000 nucleotides, at least 2500 nucleotides, at least 3000 nucleotides or at least 3500 nucleotides. In a particularly preferred example an isolated polynucleotide has a length of at least 87 nucleotides or at least 100 nucleotides.

The isolated polynucleotide has a length of between at least 93 and 9000 nucleotides, preferably at least 93 and 8000 nucleotides, more preferably between at least 93 and 7000 nucleotides, more preferably between at least 93 and 6000 nucleotides, more preferably between at least 93 and 5000 nucleotides, more preferably between at least 93 and 4000 nucleotides, more preferably between at least 93 and 3000 nucleotides, more preferably between at least 93 and 2000 nucleotides, more preferably between at least 93 and 1000 nucleotides or even more preferably between at least 93 and 500 nucleotides.

An isolated polynucleotide has a length of at least 24 nucleotides, at least 50 nucleotides, at least 87 nucleotides, at least 93 nucleotides, at least 100 nucleotides, at least 200 nucleotides, at least 500 nucleotides, at least 800 nucleotides, at least 1000 nucleotides, at least 1500 nucleotides, at least 2000 nucleotides, at least 2500 nucleotides, at least 3000 nucleotides or at least 3500 nucleotides. In a particularly preferred example the isolated polynucleotide has a length of at least 24, at least 87 or at least 93 nucleotides.

It will be apparent to the skilled person that due to the degeneracy of the genetic code a given polypeptide may be encoded by different nucleotide sequences.

In a preferred embodiment the isolated polynucleotide according to the invention comprises or consists of a sequence which exhibits at least 80% identity to the sequence according to SEQ ID NO.:4.

In a further preferred example the polynucleotide comprises or consists of a sequences which exhibits at least 85%, preferably at least 90%, more preferably at least 91 %, even more preferably at least 92%, even more preferably at least 93%, even more preferably at least 94%, even more preferably at least 95%, even more preferably at least 96%, even more preferably at least 97%, even more preferably at least 98% or even more preferably at least 99% identity to the sequence according to SEQ ID NO.:4. The polynucleotide comprises or consists of a sequence which exhibits at least 85%, preferably at least 90%, more preferably at least 95% or even more preferably at least 98% identity to the sequence according to SEQ ID NO.:4.

The polynucleotide comprises or consists of a sequence which exhibits at least 80%, at least 85%, preferably at least 90%, more preferably at least 91%, even more preferably at least 92%, even more preferably at least 93%, even more preferably at least 94%, even more preferably at least 95%, even more preferably at least 96%, even more preferably at least 97%, even more preferably at least 98% or even more preferably at least 99% identity to the sequences according to SEQ ID NO.:4.

In a particularly preferred embodiment the isolated polynucleotide according to the invention comprises or consists of sequence which exhibits 100% identity to the sequence according to SEQ ID NO.:4.

An isolated polynucleotide may be a single or double stranded RNA or DNA molecule.

The isolated polynucleotide according to the invention is inserted into an expression vector. The expression vector may be a prokaryotic or cukaryotic expression vector such as e.g. a plasmid, a miniclromosome, a cosmic, a bacterial phage, a retroviral vector or any other vector known to the skilled person. The skilled person will be familiar with how to select an appropriate vector according to the specific need.
The present invention thus relates to an expression vector comprising a polynucleotide as claimed in 3) and 4) of claim 1.

As used herein the term "hybridize" or "hybridizes" refers to the hybridization of a first to a second polynucleotide. To determine, if two polynucleotide hybridize to each other, the skilled person will preferably conduct hybridization experiments in vitro under moderate or stringent hybridization conditions. Hybridization assays and conditions arc known to those skilled in the art and can be found, for example, in Current Protocols in Molecular Biology, John Wiley & Sons, N. Y., 6.3.1-6.3.6, 1991. Stringent conditions may e.g. be conditions in which hybridization takes place in 6X sodium chloride/sodium citrate (SSC) at 45°C, followed by a wash in 0.2 X SSC, 0.1 % SDS at 65°C.

The present invention relates to a pharmaccutical composition comprising a compound capable of inhibiting the activity and/or the expression of the CCR6 receptor as claimed in claim 1.

A pharmaceutical composition according to the invention can be administered orally, for example in the form of pills, tablets, lacquered tablets, sugar-coated tablets, granules, hard and soft gelatin capsules, aqueous, alcoholic or oily solution, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures.

In a preferred example a pharmaceutical composition is administered parenterally, e.g. in form of solutions for injection or infusion, orally, e.g. in the form of tablet, pill, lozenge or capsule or via inhalation.

For the production of pills, tablets, sugar-coated tablets and hard gelatin capsules for example, lactose, starch, for example maize starch, or starch derivatives, talc, stearic acid or its salts, etc may be used. Carriers for soft gelatin capsules and suppositories arc, for example, fats, waxes, semisolid and liquid polyols, natural or hardened oils, etc. Suitable carriers for the preparation of solutions, for example of solutions for injection, or of emulsions or syrups arc, for example, water, physiological sodium chloride solution, alcohols such as ethanol, glycerol, polyols, sucrose, invert sugar, glucose, mannitol, vegetable oils, etc.

In some example the pharmaceutical compositions also contains additivcs, such as for example fillers, disintegrants, binders, lubricant, wetting agents, stabilizers, emulsifiers, dispersants, preservatives, sweeteners, colorants, flavorings, aromatizcrs, thickeners, diluents, buffer substances, solvents, solubilizers, agents for achieving a depot effect, salts for altering the osmotic pressure, coating agents or antioxidants.

Examples of suitable excipients for the various different forms of pharmaceutical compositions described herein may e.g. be found in the "Handbook of Pharmaceutical Excipicnts", 2nd Edition, (1994), Editcd by A Wade and PJ Weller. In another preferred embodiment, a pharmaceutical composition according to the invention comprises a further active compound suitable for the treatment or prevention of an interstitial lung disease and/or cancer.

Examples of further active compound suitable for the treatment of an interstitial lung diseases arc e.g. anti-inflammatory drugs such as e.g. prednisone or other corticosteroids, azathioprine, methotrexate, mycophenolate or cyclophosphamide, antioxidants, such as e.g. acetylcysteine anti-fibrotic agents, such as e.g. bosentan or pirfenidone, minocycline, sildenafil, thalidomide, anti-TNF antibodies, such as e.g. lnfliximab; etanercept, interferon gamma, anti-IL-13 antibodies, endothelin inhibitors, Zilleuton, anticoagulant, macrolides, phosphodiesterase (PDE) 4 inhibitors, such as e.g. roflumilast, Aviptadil, alpha-melanocyte-stimulating hormone (alpha -MSH), tyrosine kinase inhibitors, such as e.g. imatinib, dasatinib and nilotinib.

The further active compound suitable for the treatment of cancer is preferably a chemotherapeutic agent. Chemotherapeutic agents suitable for the treatment of cancer are well known to the skilled person. Examples of chemotherapeutic agents are e.g. temozolomide, adriamycin, doxorubicin, epirubicin, 5-fluorouracil, cytosine arabinoside ("Ara-C"), cyclophosphamide, thiotepa, busulfan, cytoxin, taxoids, e.g., paclitaxel, toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin,mitomycins, melphalan and other related nitrogen mustards and hormonal agents that act to regulate or inhibit hormone action on tumors such as tamoxifen and onapristonc.

The pharmaceutical composition may comprise one or more than one further active compound suitable for the treatment or prevention of an interstitial lung disease and/or one or more than one further active compound suitable for the treatment or prevention of cancer, In a preferred example the pharmaceutical composition comprises 1,2,3,4,5,6,7,8,9,10 or more than 10 further active compounds suitable for the treatment of prevention of an interstitial lung disease and/or 1,2,3,4,5,6,7,8,9,10 or more than 10 further active compound suitable for the treatment of cancer.

One or more separate compositions comprising an active compound suitable for the treatment or prevention of an interstitial lung disease and/or an active compound suitable for the treatment or prevention of cancer may be administered to a subject, preferably a human subject, in combination with a pharmaceutical composition according to the invention comprising a compound capable of inhibiting the activity and/or the expression of the CCR6 receptor.

In a further aspect the present invention relates to a pharmaceutical composition according to the invention for use in the treatment of an interstitial lung disease and/or cancer.

In one preferred example of the pharmaceutical composition according to the invention the interstitial lung disease is selected from the group consisting of diffuse parenchymal lung disease of known cause (preferably collagen vascular disease or environmental or drug related diffuse parenchymal lung disease), granulomatous diffuse parenchymal lung disease (preferably sarcoidosis) and other forms of diffuse parenchymal lung disease (preferably lymphangiolciomyomatosis (LAM), pulmonary Langerhans cell histiocytosislhistiocytasis X (HX) or eosinophilic pneumonia).

In a particularly preferred embodiment of the pharmaceutical composition according to the invention the interstitial lung disease is an idiopathic interstitial pneumonia.

In a further preferred embodiment of the pharmaceutical composition according to the invention the interstitial lung disease is selected from the group consisting of idiopathic pulmonary fibrosis, nonspecific interstitial pneumonia, cryptogenic organizing pneumonia, respiratory bronchiolitis-associated interstitial lung disease, desquamtive interstitial pneumonia, acute interstitial pneumonia and lympltocytic interstitial pneumonia. In a particularly preferred embodiment of the pharmaceutical composition according to the invention the interstitial lung disease is idiopathic pulmonary fibrosis. For an overview on the classification of interstitial lung diseases the skilled person may e.g. refer to American Thoracic Society/European Respiratory Society International Multidisciplinary Consensus Classification of the Idiopathic Interstitial Pneumonias, American Thoracic Society; European Respiratory Society, Am J Respir Crit Care Med. 2002 Jan 15;165(2):277-304.

Interstitial lung disease may be caused by a condition selected from the group consisting of scleroderma lung disease, sarcoidosis, hypersensitivity pneumonitis, rheumatoid arthritis, lupus crythematosus and asbestosis.

In another preferred example of the pharmaccutical composition according to the invention the cancer is selected from the group consisting of lung cancer, breast cancer, pancreatic cancer, prostate cancer, neuroblastoma, melanoma, brain cancer, kidney cancer, bladder cancer, ovarian cancer, blood cancer and colon cancer. In a norther preferred embodiment of the pharmaceutical composition according to the invention the cancer is selected from the group consisting of adenorcarcinoma, preferably adenocarcinoma of the lung, pleuramesothelioma, colorectal carcinoma, prostate carcinoma, mamma carcinoma, renal cell carcinoma, hepatocellular carcinoma, Non-Hodgkin-Lymphoma and Hodgkin-Lymphoma.

In a particularly preferred embodiment of the pharmaceutical composition according to the invention the cancer is lung cancer, most preferably adenocarcinoma of the lung, or pleural mesothelioma. In a most preferred embodiment the cancer is adenocarcinoma of the lung.

The present invention will now be described with respect to some of its specific examples.

### EXAMPLES

### Example 1 - Materials and Methods

### Phase display library

For the identification of a CCL18-binding motif an established phage-dispiay library was used (1).

### Cells and cell lines

Fibroblast lines were established either from surgical material from pneum-ectomies or lobe-ectomies from patients suffering from various tumours or from remaining material obtained from fibrotic lungs by video -assistcd thoracoscopies (VATS). The patients suffered ether from adenocarcinoma of the lung, non-small cell carcinoma or squamous carcinoma. Fibrosis resulted from idiopathic pulmonary fibrosis (UIP), non-specific interstitial pneumonia, sarcoidosis, hypersensitivity pneumonitis, pneumoconiosis or systemic scleroderma. The tissue was cut in small peaces (app. 0.5 cm edge length) and placed in 6-well plates containing 1 ml Quantum 333 (PAA, Pasching, Austria) with 1% penicillin/streptomycin. Outgrowing fibroblasts were harvested when they reached approximately 80% confluence by trypsinisation, cultured in 75cm2 cell culture flasks (NUNC Thermo Fisher, Roskilde, Denmark) in Quantum 333 and subsequently split (1:3 to 1:5). The established lines were enrolled in the studies from passages 3 to 8.

If not otherwise mentioned cells were stimulated with 10 ng/ml human recombinant CCL18, 2.5 ng/ml TGFβ or 1 ng/ml TNFα either alone or in combination.

AECII were isolated as described previously (2). In brief, macroscopically tumor-free lung tissue was first sliced and slices were washed three times at 4°C in phosphate-buffered saline (PBS) and then digested in sterile dispase solution (2,5mg dispasc II (Invitrogen GmbH, Karlsruhe, Germany) ml and 50µg/ml DNasc I (Roche Diagnostics, Mannheim, Germany)) at 37°C for 60 minutes. After dispase digestion, the slices were thoroughly pipetted for several minutes using a 10ml pipette with a wide inlet. Crude tissue and cell suspensions were filtered through nylon gauze with meshes of 50 and 20 µm. The cell suspension was then layered onto a density gradient solution (PAN Biotech GmbH, Aidcnbach, Germany) and centrifuged at 800×g for 20 min.

The cells from interphase were washed and incubated in 100mm Petri dishes (max. 6x107cells/dish) in RPMI (Invitrogen, Karlsruhe, Germany) with 10% fetal calf serum (FCS) (PAA Laboratories GmbH, Cölbe, Germany) and 1% penicillin/streptomycin (Biochrom AG, Berlin, Germany) at 37°C in humidified incubator (5%CO2, 37°C) for 15, 20 and if possible 30 minutes to remove adherent (mostly alveolar macrophages, monocytes and fibroblasts) from non-adherent cells.

### Flow cytometry

Surface expression of CCR6 of fibroblast lines was estimated using an FITC-labelled anti-human CCR6 antibody (R&D Systems, Minneapolis, MN). CCR6 expression of RLE-6TN was analysed using a polyclonal goat anti-CCR6 antibody (CKR6/C20, Santa Cruz Biotechnology, Santa Cruz, CA) and a FITC labelled mouse-anti-goat antibody (DAKO, Glostrup, Denmark).

Surface expression of CCR6 of tumor cell lines was estimated using a PE-labelled anti-human CCR6 antibody (R&D Systems, Minneapolis, MN) and measured with a FACS Calibur (Beckton Dickinson, Heidelberg, FRG).

Human lung adenocarcinoma cells and pleural mesothelioma cells were a generous gift from Prof. HH Fiebig, Oncotest, Freiburg. The cells were cultured in RPM11640 containing 10% FCS and 100U/ml streptomycin and penicillin. Cultures were splitted 1:3 when the cultures reached 90% confluence.

### Immunohistochemistry

Immunohistochemistry was performer as described (3,4) using anti-human CCR6 mAb (clone 53103, isotypc mouse IgG2b; R&D Systems Europe, UK) and a peroxidase-labeled streptavidin-biotin technique (DAKO LSAB2 System; DakoCytomation, Germany). Counterstain was performed with Mayer's hemalum. For negative control included in every staining series, sections were stained without primary antibody.

### Analysis of CCR6 expression in human lung carcinoma

Freshly isolated tumour tissue from adenocarcinoma of the lung was cut in pieces of not more than 1x1x0.5 cm edge length. The tissue was stabilised using HOPE stabilizing solution (DCS, Hamburg, FRG) at 4°C and paraffin embedded.

Tissue slices were deparaffinized and stained for CCR6 expression by peroxides technique (5) using a commercially available antibody (R&D Systems, Wiesbaden, FRO).

### FGF2 ELISA

To estimate CCL18-induced FGF2 release fibroblasts were harvested, counted and seeded 300.000 cells per well in a 6-wcll cell culture plate in Quantum 333. Cells were allowed to attach over night and the medium was replaced with I ml of DMEM plus 10% FCS. Cells were either left un-stimulated or were stimulated with 10ng/ml of human recombinant CCL18. To block CCR6/CCL18 interaction a blocking antibody against CCR6 (R&D systems, Wiesbaden, FRAG) or an irrelevant antibody (mouse anti human IgG, R&D Systems) was added to a final concentration of 20µg/ml in parallel cultures. After 24h of culture the supernatant was harvested and stored at -80°C until FGF2 determination.

FGF2 concentrations were measured using an ELISA development kit (R&D) and performed as suggested by the supplier.

### Real-time PCR

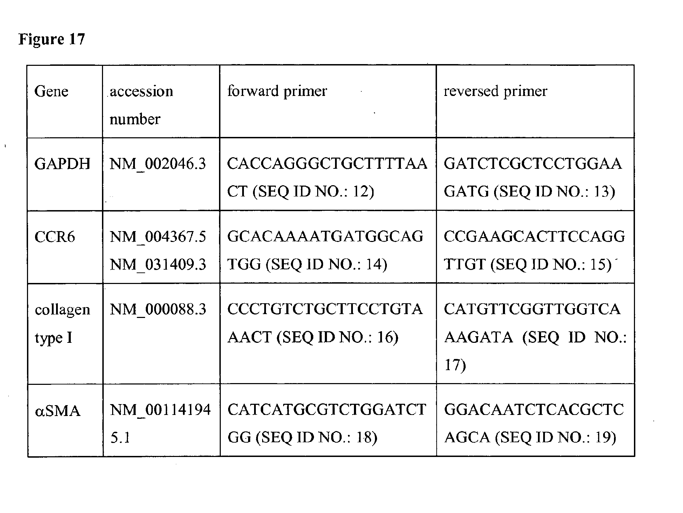

After the indicated culture period total RNA was extracted using TRIzol Reagent according to the manufacturer's, protocol (Invitrogen, Karlsruhe, Germany). Total RNA was reverse-transcribed with StrataScript RT (Stratagene, CA) using oligo (dT)12-18 primer to produce cDNA according to the manufacturer's protocol. Specific primers for human CCR6, collagen type I, alpha-smooth muscle actin and GAPDH were designed using Primer3 software (Whitehead Institute for Biomedical Research, Cambridge, USA; http://frodo.wi.m it.edu/cgi-bin/primer3/primer3_www.cgi), Amplifyl.2 software (University of Wisconsin, USA; http://engels.genetics.wise.edu/amplify) using LocusLink and GenBank databases (National Center for Biotechnology Information; http://www.ncbi.nlm.nih.gov/LocusLink/index.html). Accession code numbers for the nucleotide sequences used to generate the respective primers and the primer sequences arc depicted in figure 17.

All primers were intron-spanning and synthesized by MWG-Biotech (MWG-Biotech AG, Germany). Real time PCR was performed with the iQ SYBR Green SuperMix, iCycler thermocycler, and iCycler iQ 3.0 software (Bio-Rad Laboratories GmbH, Germany) according to the manufacturer's protocol. To control for specificity of the amplification products, a melting curve analysis was performed. No amplification of nonspecific products was observed in any of the reactions. A threshold cycle value (Ct) was calculated and used to compute the relative level of specific mRNA by the following formula: "relative expression = 2^{(Ct GAPDH-Ct CCL18)}x10,000" for each CDNA sample.

### Western Blot

After the indicated culture period the cells were washed once in PBS and lysed in ice cold lysis buffer. Whole cell lysates were boiled at 93°C for 5 minutes in equal volumes of loading buffer (0,5M Tris-HCl pH 6.8, 2% SDS, 0, 05% bromphenolblue, 20 % 2-mercaptoethanol, 10% Glycerol).

All samples were subjected to 12% sodium dodecylsulfate-PAGE, seperated by clectrophoresis and transferred to a polyvinylidene difluoride membrane (PVDF). After blocking for 2h in Tris-buffered saline (TBS) containing 5% non-fat dry milk, the membranes were incubated with primary antibody (rabbit-anti-collagen I (Rockland, Glibertsville PA); anti αSMA (rabbit-anti-αSMA (Abcam, Cambridge, MA); anti vimcntin (Santa Cruz); anti CCR6: CKR6/C20, Santa Cruz) diluted 1:700 with TBS at 4°C overnight. Visualization was performed using appropriate secondary antibodies labeled with IRDye 800CW or IRDye 700CW (Li-COR Bioscience, Bad Homburg, Germany) diluted 1:10 000-1:20000 for 2 h and scanned using Odyssey system (Li-COR Biosience) according to the manufactures instructions.

### Analysis of CCR6 expression by PBMNCs in presence or absence of PHA

Human peripheral blood mononuclear cells were isolated from venous puncture blood by density centrifugation, washed and counted. Cells were adjusted to 1x10⁶ cells per ml in complete culture medium (RPMI1640 with 10% foetal calf serum and 100 U penicillin/streptomycin) and cultured for 24 h either in the absence or presence of 10µg/ml phytohemeagglutinin (PHA). The percentage of CCR6-positive cells was measured using FITC-labelled anti-CCR6 antibodies (R&D Systems, Wiesbaden FRAG) and counted in an FACS calibur (Becton Dickinson, Heidelberg, FRG).

### Inhibition of CCR6 down-regulation

Freshly isolated mononuclear cells were incubated for 20 minutes with 10 ng/ml of CCL18, 100ng/ml of the inhibitor peptide PS-AU-105 (polypeptide according to SEQ ID NO.:1), a combination of both or were left untreated. The incubation was performed in RPMI1640 with 10% fetal calf serum and 100 U/ml Streptomycin/Penicillin at 37°C. After the incubation, the cells were centrifugated and the supernatant was discarded. The cells were fixed with 4% paraformaldehyde for 5 minutes on ice and washed twice in PBS containing 1% FCS. After fixation the cells were stained using a fluorescein-conjugated antibody against CCR6 (R&D systems, Wiesbaden, FRG) and analyzed by flow cytometry (FACScalibur and Quantiquest; both Beckon Dickinson, Franklin Lakes, USA).

### Statistics

All statistics were performed using nonparametric statistics. Pair wise analysis of increase or inhibition was carried out using Wilcoxon-signed rank analysis; unpaired groups were analysed using Mann-Whitney U Test. Values of p <0.05 were considered significant. Data arc presented using Box-Plots. In this case median is given as line in a rectangutar indicating the 25 and 75 percentile. The 5 and 95 percentile are indicated as lines beneath or above the rectangular, values beyond or above the 5 and 95 percentile arc illustrated as dot.

### Example 2 - Identification of a CCL18-binding peptide using phage display

After 3 circles of binding of the phages of the page library (as described in example 1) to plate-bound human CCL18, phage-infected E. coli were plated on agar and 20 colonies were picked, expanded and DNA was isolated for sequencing. Some of the sequences were not informative or coded for non-human gene targets, however, one sequence with a clear relation to the CC-chemokine receptor 6 (CCR6) was identified.

### Example 3 - Analysis of CCR6 expression on primary human fibroblast line

### PCR

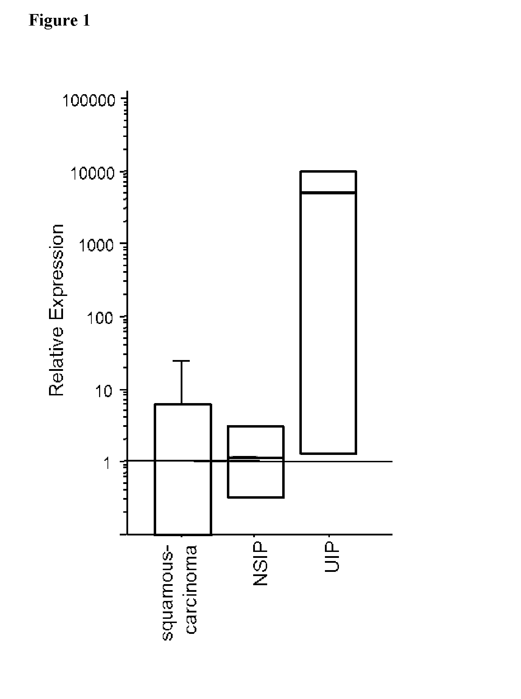

Expression studies using primers for CCR6 revealed a high variability of CCR6 mRNA expression. Highest expression was found in fibroblast lines derived from lung of patients suffering from usual interstial pneumonia (UIP). Lines derived from patients suffering from non-specific interstitial pneumonia (NSIP) or squamous carcinoma expressed only marginal levels of CCR6 mRNA (Fig. 1).

### FACS

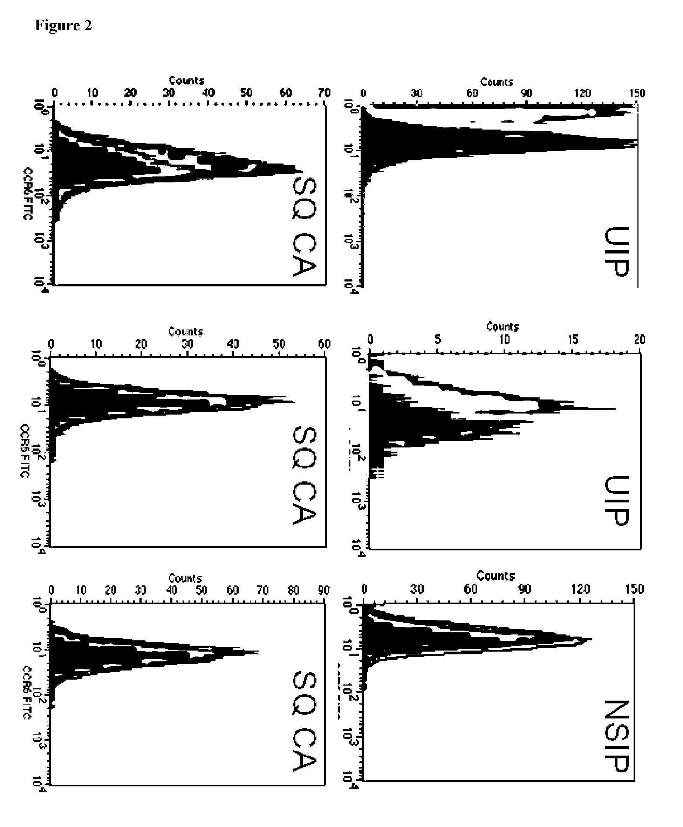

Flow-cytometric analyses of the established fibroblast lines revealed detectable CCR6 expression only on fibroblast lines derived from patients suffering from UIP. Lines generated from lunges of patients suffering from squamous carcinoma or NSIP do not express detectable CCR6 on their surface (Fig. 2). Increased CCR6 expression of fibroblasts from fibrotic Lungs remained increased throughout all passages (data not shown).

### Immunohistochemistry

Immunohistochemical studies on tissue sections taken from lungs of patients with IPF/UIP did not reveal CCR6 expression in normal lung tissue (Fig. 3A). In fibrotic lungs CCR6 expression was detected on the apical surface of alveolar epithelial cells (Fig. 3B) and on fibroblasts (Fig. 3C).

### Example 4 - Functional Analysis of CCR6

### FGF2 expression

Fibroblasts derived from non-fibrotic lungs released only marginal levels af FGF2. CCL18 stimulates FGF2 release in these cells only marginally. In contrast, non-stimulated fibroblasts derived from fibrotic lungs release increased levels of FGF2 (p<0.005 compared with controls) which is further up-regulated by CCL18 (p<0.45, Fig. 4).

Blockade of CCR6 by a blocking antibody exerts no effect on CCL18 stimulated FGF2 release by non-fibrotic fibroblast lines. In fibroblast derived from fibrotic lungs blockage of CCR6 significantly diminished CCL18 induced FGF2 up-regulation (p<0.05).

### Collagen expression

CCL18 has been reported to induce the expression of collagen and αSMA (7, 18). It was thus determined whether the induction of these molecules is also CCR6 dependent. As shown in Fig. 5 (upper panel) CCL18 up-regulates collage I expression in three of four cell lines analysed. This induction was inhibited in all three CCL18 reactive cell lines by the blockade of CCR6 using a blocking antibody. The same result was received for the CCL18 induced up-regulation of αSMA. The same cell line which failed to increase collagen I expression by CCL 18 stimulation did also not react in case of αSMA (Fig. 5, lower panel).

### Examples 5 - Induction of Epithelial-Mesenchymal-Transition (EMT) to myo-fibroblasts by CCL18 is CCR6-dependent

### EMT of the rat alveolar epithelial cell line RLE-6TN

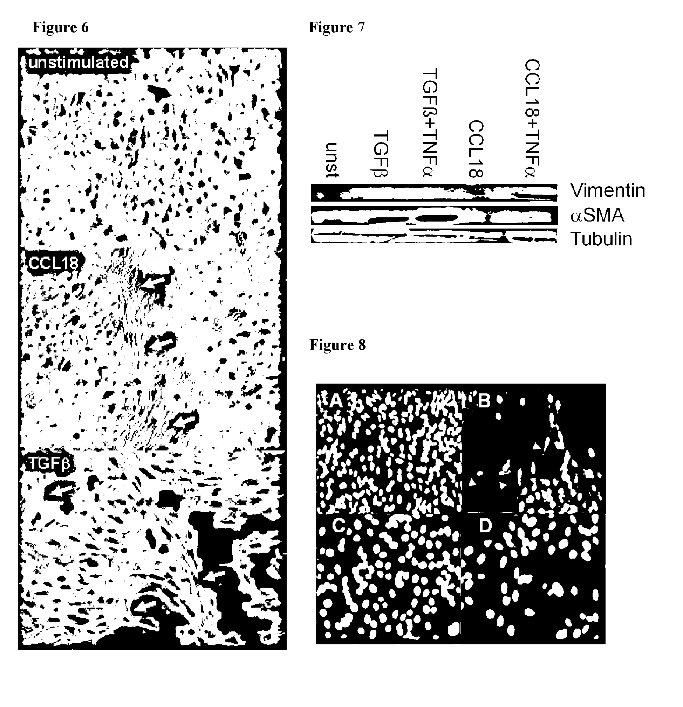

The rat alveolar epithelial cell line RLE-6TN discloses the typical cuboid cell form of epithelial cell (Fig. 6 top). Culture of these cells for six days in the presence of CCL18 induces a differentiation into spindle-shaped, fibroblast-like cells (Fig. 6 mid, see arrows) in some areas. This differentiation is more pronounced in the presence of TGFβ (Fig. 6 bottom, see arrows) as almost all cells disclose a spindle-shaped, fibroblast-like phenotype. This indicates that CCL 18 induce at least partially EMT in RLE-6TN cells.
Western Blot analyses of RLE-6TN after EMT induced either by CCL18 or TGFβ revealed expression of vimentin after culture in the presence of CCL18 which is enhanced by TNFα Figure 7). Only a faint expression of αSMA was visible in both conditions. TGFβ induced a strong expression of αSMA but failed to induce the expression of vimentin.

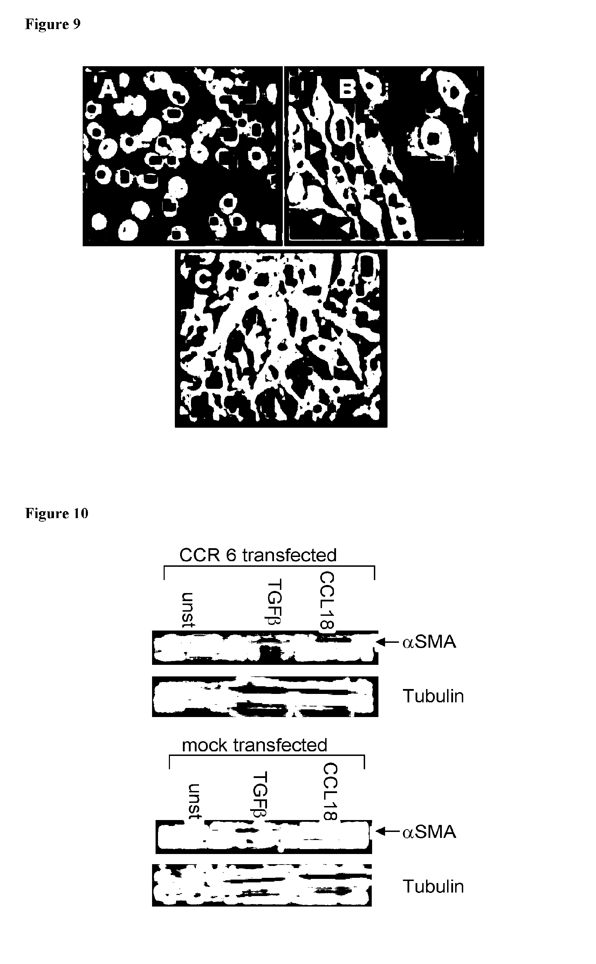

Fluorescence microscopy demonstrated that stimulation with TGFβ indeed induces αSMA expression in RLE-6TN cells (Fig. 8B) whereas CCL18 or CCL18/TNFα did not (Fig. 8C and D). In contrast, all stimulations induced the expression of CD90 (Fig. 9B and C) although CD90 expression was more intense using CCL18. Quantitative real-time PCR of RLE-6TN revealed a faint expression of rat CCR6. However, CCR6 protein analysis using Western Blot or flow cytometry could not demonstrate the expression of CCR6 by RLE-6TN (data not shown).Thc cells were therefore transiently transacted with a commercially available plasmid containing a DNA sequence coding for human CCR6. After transfection, stimulation with CCL18 induced αSMA in CCR6 transfected cells, but not in mock transfected cells. In contrast, TGFβ induced αSMA expression in both cell types (Figure 10).

### EMT of human primary alveolar epithelial cells

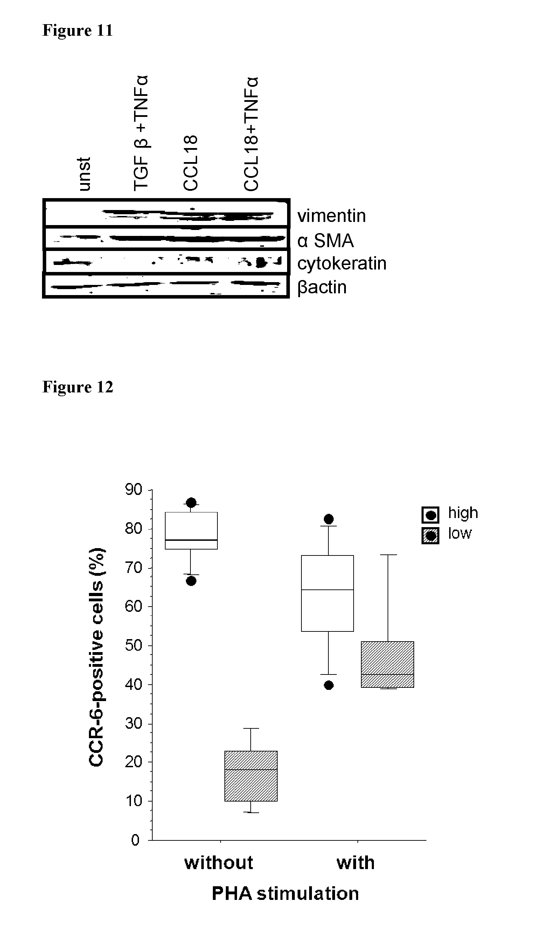

Stimulation of isolated human primary alveolar epithelial cells type II stimulated with either TGFβ-TNFα or CCL18 with or without TNFα also induces EMT as demonstrated by the induction of vimentin and αSMA expression and the reduced expression of cytokeratin (Fig. 11).

### Example 6 - Inhibition of CCL18-induced down regulation of CCR6 expression on PBMNCs

Isolated human pcripherat blood manonuclcar cells (PBMNC) were grouped according to their basal percentage of CCR16 cells. Culture of PBMNC without any activation does not alter the percentage of CCR6⁺ cells. In contrast, activation with PHA increases CCR6⁺ percentage in preparations with low initial CCR6⁺ proportion (Fig. 12 shaded bars), but decreases it in preparations with high CCR6⁺ proportion (Fig. 12 white bars).

Incubation of isolated, CCR6⁺ rich PBMNCs with CCL18 for 20 minutes resulted in a decrease in the percentage of CCR6⁺ cells (Fig. 13) possibly due to internalization of the CCR6 receptor. This CCL18 induced redaction of CCR6 expression is diminished in the presence of the inhibitor peptide PS-AU-105 (polypeptide according to SEQ ID No.: 1). Fig. 14 demonstrates a clear sub-population of CCR6⁺ cells in non-stimulated (pos. control) culture. Stimulation with CCL18 diminishes this subpopulation as demonstrated by a lowered second peak (CCL18 only). CCL18 stimulation in the presences of the inhibitor peptide PS-AU-105 (polypeptide according to SEQ ID No.: 1) fails to down-regulate the CCR6⁺ sub-population. The inhibitor atone exhibits no effect (inhibitor only).

### Example 7- Analysis of CCR6 expression in human lung carcinoma

### Immunohistochemistry

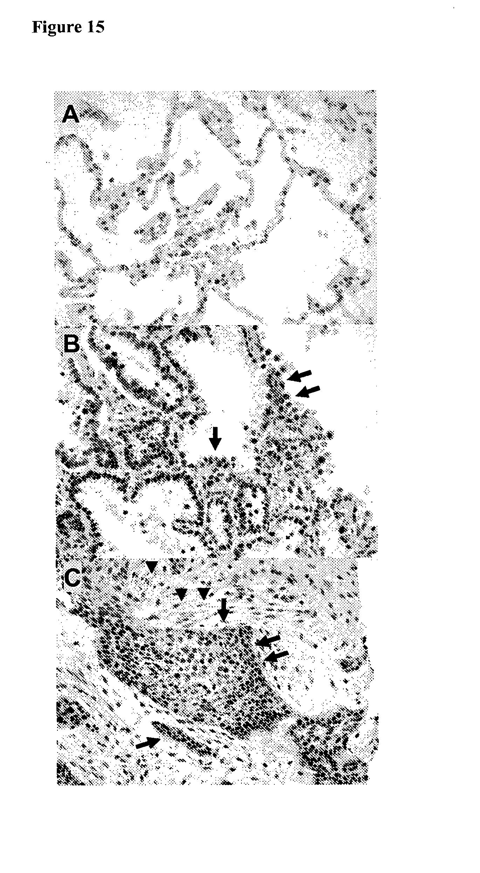

Analysis of control lung revealed no CCR6 staining (Fig. 15A). In contrast, tumour cells disclosed a clear CCR6 expression (Fig. 15 B and C, arrows). In addition, some fibroblast also disclosed a faint CCR6 expression (Fig. 15 C, arrow heads).

### FACS

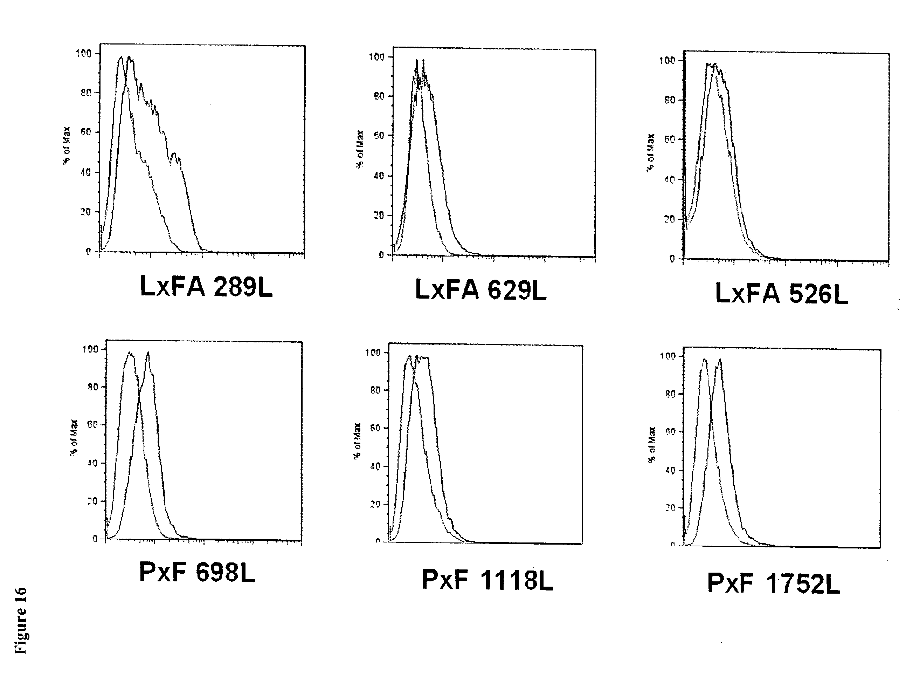

FACS analysis revealed a clear cut CCR6 expression in two out of three adenocarcinoma cell lines (Fig. 16 upper panel) and all pleural mesothelioma cell lines (Fig. 16 lower panel). The lung adenocarcinoma cell line LxFA 526L discloses marginal CCR6 expression.

### LITERATURE

1. Trepel, M., Arap, W., and Pasqualini, R. 2002. In vivo phage display and vascutar heterogeneity: implications for targeted medicine. Curr Opin Chem Biol 6:399-404.
2. Pechkovsky, D.V., Zissel, G., Ziegenhagen, M.W., Einhaus, M., Taube, C., Rabe, K.F., Magnussen, H., Papadopoulos, T., Schlaak, M., and Muller-Quernheim, J. 2000. Effect of proinflammatory cytokines on interleukin-8 mRNA expression and protein production by isolated human alveolar epithelial cells type II in primary culture. Eur Cytokine Netw 11:618-625.
3. Goldmann, T., Wicdorn, K.H., Kuhl, H., Olert, J., Branscheid, D., Pechkovsky, D., Zissel, G., Galle, J., Muller-Quernheim, J., and Vollmer, E. 2002. Assessment of transcriptional gene activity in situ by application of HOPE-fixed, paraffin-embedded tissues. Pathol Res Pract 198:91-95.
4. Pcchkovsky, D.V., Zissel, G., Goldmann, T., Einhaus, M., Taube, C., Magnussen, H., Schlaak, M., and Muller-Quernheim, J. 2002. Pattern of NOS2 and NOS3 mRNA expression in human A549 cells and primary cultured AEC II. Am J Physiol Lung Cell Mol Physiol 282:L684-692.
5. Droemann, D., D. Albrecht, J. Gerdes, A.J. Ulmer, D. Branscheid, E. Vollmer, K. Dalhoff, P. Zabel, and T. Goldmann, Human lung cancer cells express functionally active Toll-like receptor 9. Rcspir Res, 2005. 6(1): p. 1.

## Claims

1. A pharmaceutical composition comprising a compound capable of inhibiting the activity and/or the expression of the CCR6 receptor for use as medicament, wherein the compound capable of inhibiting the activity and/or the expression of the CCR6 receptor is selected from the group consisting of the isolated polypeptide according to 1) or 2) and the expression vector according to 3) or 4), wherein
1) is an isolated polypeptide consisting of an amino acid sequence selected from the group consisting of:
(a) an amino acid sequence which exhibits at least 80% identity to the sequence according to SEQ ID NO.: 1; and
(b) a fragment of the amino acid sequence according to (a);
wherein said isolated polypeptide is capable of binding to and inhibiting the activity of the CCR6 receptor;
2) is the isolated polypeptide according to 1), wherein the amino acid sequence according to (a) comprises at least 8 consecutive amino acids of the sequence according to SEQ ID NO.: 1;
3) is an expression vector comprising an isolated polynucleotide encoding the polypeptide according to 1) or 2);
4) is the expression vector comprising an isolated polynucleotide according to 3), wherein said polynucleotide comprises or consists of a sequence which exhibits at least 80% identity to the sequence according to SEQ ID NO.: 4.

2. A pharmaceutical composition according to claim 1 for use in the treatment of an interstitial lung disease and/or cancer.

3. A pharmaceutical composition for use according to claim 2, wherein the composition comprises a further active compound suitable for the treatment of an interstitial lung disease and/or cancer.

4. The pharmaceutical composition for use according to claim 2 or 3, wherein the interstitial lung disease is selected from the group consisting of idiopathic pulmonary fibrosis, nonspecific interstitial pneumonia, cryptogenic organizing pneumonia, respiratory bronchiolitis-associated interstitial lung disease, desquamative interstitial pneumonia, acute interstitial pneumonia and lymphocytic interstitial pneumonia.

5. The pharmaceutical composition for use according to claim 2 or 3, wherein the cancer is selected from the group consisting of adenocarcinoma, preferably adenocarcinoma of the lung, pleuramesothelioma, colorectal carcinoma, prostate carcinoma, mamma carcinoma, renal cell carcinoma, hepatocellular carcinoma, Non-Hodgkin-Lymphoma and Hodgkin-Lymphoma.

## Patentansprüche

1. Eine pharmazeutische Zusammensetzung umfassend eine Verbindung, die in der Lage ist, die Aktivität und/oder die Expression des CCR6 Rezeptors zu inhibieren, zur Verwendung als Medikament, wobei die Verbindung, die in der Lage ist, die Aktivität und/oder die Expression des CCR6 Rezeptors zu inhibieren, ausgewählt ist aus der Gruppe bestehend aus dem isolierten Polypeptid gemäß 1) oder 2) und dem Expressionsvektor gemäß 3) oder 4), wobei
1) ein isoliertes Polypeptid bestehend aus einer Aminosäuresequenz ist, ausgewählt aus der Gruppe bestehend aus:
(a) einer Aminosäuresequenz, welche mindestens 80% Identität mit der Sequenz der SEQ ID NO.: 1 aufweist; und
(b) einem Fragment der Aminosäuresequenz gemäß (a);
wobei besagtes isoliertes Polypeptid in der Lage ist, an den CCR6 Rezeptor zu binden und dessen Aktivität zu inhibieren;
2) das isolierte Polypeptid gemäß 1) ist, wobei die Aminosäuresequenz gemäß (a) mindestens 8 konsekutive Aminosäuren der Sequenz der SEQ ID NO.: 1 umfasst;
3) ein Expressionsvektor umfassend ein isoliertes Polynukleotid ist, der für das Polypeptid gemäß 1) oder 2) kodiert;
4) der Expressionsvektor umfassend das isolierte Polynukleotid gemäß 3) ist, wobei besagtes Polynukleotid eine Sequenz umfasst oder aus einer Sequenz besteht, welche mindestens 80% Identität mit der Sequenz der SEQ I NO.: 4 aufweist.

2. Eine pharmazeutische Zusammensetzung gemäß Anspruch 1 zur Verwendung bei der Behandlung einer interstitiellen Lungenerkrankung und/oder Krebs.

3. Eine pharmazeutische Zusammensetzung zur Verwendung gemäß Anspruch 2, wobei die Zusammensetzung einen weiteren Wirkstoff umfasst, welcher sich zur Behandlung von interstitieller Lungenerkrankung und/oder Krebs eignet.

4. Die pharmazeutische Zusammensetzung zur Verwendung gemäß Anspruch 2 oder 3, wobei die interstitielle Lungenerkrankung ausgewählt ist aus der Gruppe bestehend aus idiopathischer Lungenfibrose, nicht-spezifischer interstitieller Pneumonie, kryptogen organisierter Pneumonie, Atemwegs-Bronchiolitis assoziierter interstitieller Lungenkrankheit, desquamativer interstitieller Pneumonie, akuter interstitieller Pneumonie und lymphozytischer interstitieller Pneumonie.

5. Die pharmazeutische Zusammensetzung zur Verwendung gemäß Anspruch 2 und 3, wobei der Krebs ausgewählt ist aus der Gruppe bestehend aus einem Adenokarzinom, vorzugsweise einem Adenokarzinom der Lunge, Pleuramesotheliom, Dickdarmkrebs, Prostatakrebs, Brustkrebs, Nierenzellkarzinom, hepatozellulärem Karzinom, Non-Hodgkin Lymphom und Hodgkin Lymphom.

## Revendications

1. Composition pharmaceutique comprenant un composé capable d'inhiber l'activité et/ou l'expression du récepteur CCR6 pour une utilisation comme médicament, dans laquelle le composé capable d'inhiber l'activité et/ou l'expression du récepteur CCR6 est choisi dans le groupe consistant en le polypeptide isolé suivant 1) ou 2) et le vecteur d'expression suivant 3) ou 4), où :
1) est un polypeptide isolé consistant en une séquence d'aminoacides choisie dans le groupe consistant en :
(a) une séquence d'aminoacides qui présente une identité d'au moins 80 % avec la séquence suivant la SEQ ID n° 1 ; et
(b) un fragment de la séquence d'aminoacides suivant (a) ;
ledit polypeptide isolé étant capable de se lier au et d'inhiber l'activité du récepteur CCR6;
2) est le polypeptide isolé suivant 1), dans lequel la séquence d'aminoacides suivant (a) comprend au moins 8 aminoacides consécutifs de la séquence suivant la SEQ ID n°1 ;
3) est un vecteur d'expression comprenant un polynucléotide isolé codant pour le polypeptide suivant 1) ou 2) ;
4) est le vecteur d'expression comprenant un polynucléotide isolé suivant 3), ledit polynucléotide comprenant ou consistant en une séquence qui présente une identité d'au moins 80 % de la séquence suivant la SEQ ID n° 4.

2. Composition pharmaceutique suivant la revendication 1, pour une utilisation dans le traitement d'une maladie pulmonaire interstitiel et/ou d'un cancer.

3. Composition pharmaceutique suivant la revendication 2, ladite composition comprenant un composé actif supplémentaire apte au traitement d'une maladie pulmonaire interstitielle et/ou d'un cancer.

4. Composition pharmaceutique pour une utilisation suivant la revendication 2 ou 3, la maladie pulmonaire interstitielle étant choisie dans le groupe consistant en la fibrose pulmonaire idiopathique, la pneumonie interstitielle non spécifique, la pneumonie organisante cryptogénique, la maladie pulmonaire interstitielle associée à la bronchiolite respiratoire, la pneumonie interstitielle desquamative, la pneumonie interstitielle aiguë et la pneumonie interstitielle lymphocytaire.

5. Composition pharmaceutique pour une utilisation suivant la revendication 2 ou 3, le cancer étant choisi dans le groupe consistant en un adénocarcinome, de préférence un adénocarcinome du poumon, un pleuramésothéliome, le carcinome colorectal, le carcinome de la prostate, le carcinome mammaire, le carcinome des cellules rénales, le carcinome hépatocellulaire, un lymphome non Hodgkinien, et le lymphome de Hodgkin.
